# EUROPEAN PATENT APPLICATION

(11) **EP 4 596 675 A1**
(43) Date of publication of application: **06.08.2025**
(21) Application number: 23871734.2
(22) Date of filing: 31.08.2023
(51) Int. Cl.: C12N 5/071, A61K 35/39, A61P 3/10, C12N 1/00

(54) **METHOD FOR PRODUCING PANCREATIC ENDODERM CELLS**

(30) Priority: 26.09.2022 JP 2022153013
(71) Applicant: Kyoto University, Kyoto-shi, Kyoto 606-8501 (JP)
(72) Inventor: OSAFUNE, Kenji, Kyoto-shi, Kyoto 606-8501 (JP); HATANO, Yu, Kyoto-shi, Kyoto 606-8501 (JP); KIMURA, Azuma, Kyoto-shi, Kyoto 606-8501 (JP)
(74) Representative: Hoffmann Eitle
(86) International application number: PCT/JP2023/031987
(87) International publication number: WO 2024/070494

(57) **Abstract**

The present invention provides: a method for producing pancreatic endoderm cells, the method comprising a step of culturing pancreatic endoderm cells in a culture medium containing a ROCK inhibitor, and KGF and/or EGF; and pancreatic endoderm cells produced by said method.

## Description

### TECHNICAL FIELD

The present invention relates to a method for producing pancreatic endoderm cells. More specifically, the present invention relates to a method for producing pancreatic endoderm cells that includes a step of culturing pancreatic endoderm cells in the presence of a ROCK inhibitor, pancreatic endoderm cells produced using this method, and the like.

### BACKGROUND ART

The pancreas functions as an exocrine gland that secretes digestive enzymes such as pancreatic lipase, trypsin, elastase, and pancreatic amylase, and an endocrine gland that secretes pancreatic hormones such as glucagon, insulin, somatostatin, and a pancreatic polypeptide (PP). In recent years, it is reported that ghrelin, which is a hormone secreted in the stomach, is also secreted by endocrine cells of the pancreas. This pancreatic hormone is produced by a cell cluster called a pancreatic islet composed mainly of four types of cells, namely an α cell, a β cell, a δ cell, and a PP cell, in the pancreas. Diabetes is a disease that is developed due to insulin insufficiency and insulin dysfunction and is difficult to completely cure once developed. Diabetes can be classified into two major types, namely type 1 diabetes (insulin-dependent diabetes) and type 2 diabetes (insulin-independent diabetes).

Type 2 diabetes is a chronic disease developed due to not only a decrease in an insulin secretion ability but also acquisition of insulin resistance and is a diabetes whose onset mechanism is lifestyle such as obesity caused by overeating or insufficient exercise, stress, and the like. Meanwhile, type 1 diabetes is a disease developed by insulin not being secreted in the body due to destruction of β cells (insulin-producing cells) caused by an autoimmune disease, viral infection, or the like. Administration of insulin is mainly conducted as symptomatic treatment. As a type 1 diabetes treatment method, it has been examined that insulin-producing cells are induced in vitro from cells derived from a patient and then the induced insulin-producing cells are transplanted to the inside of the body of the patient. The insulin-producing cells can be obtained through, for example, differentiation of pancreatic ductal epithelium-derived cells of a patient taken out to the outside of the body, and the like.

However, an invasive procedure is used to take out cells from a patient, and the number of cells obtained is insufficient. Therefore, methods for inducing differentiation of pluripotent stem cells such as induced pluripotent stem cells (iPS cells) into β-like cells necessary for cell therapy for diabetes are currently under development, and methods for inducing differentiation into β-like cells have also been actually reported (Patent Literature 1 and Non Patent Literature 1, for example). However, in these methods, as shown in FIG. 1, 6 to 7 steps (about 4 to 5 weeks) are needed to produce β-like cells from pluripotent stem cells (stepwise differentiation-inducing method).

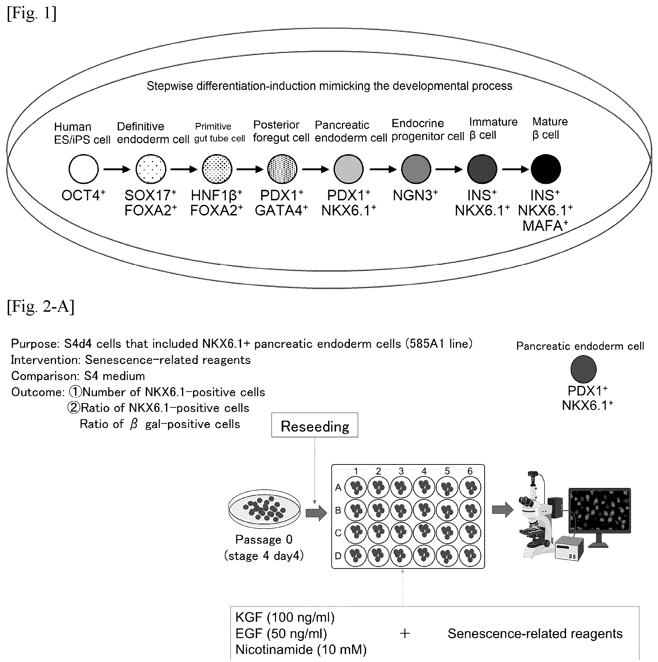

Expansion culture methods for posterior foregut cells derived from human pluripotent stem cells have also been developed (Non Patent Literature 1 and Non Patent Literature 2, for example). Non Patent Literature 1 and Non Patent Literature 2 report expansion culture methods for PDX1⁺/SOX9⁺/NKX6.1⁻ pancreatic progenitor cells or pancreatic progenitors, which correspond to posterior foregut cells in FIG. 1. However, with these methods, the PDX1⁺/SOX9⁺/NKX6.1⁻ pancreatic progenitor cells are not proliferated with high efficiency, and it is known that induction efficiency decreases when differentiation of pancreatic progenitor cells into NKX6.1-positive pancreatic endoderm cells is induced.

### Citation List

### Patent Literature

Patent Literature 1: WO 2020/059892

### Non Patent Literature

Non Patent Literature 1: Kimura et al., Cell Chemical Biology 2020 Dec 17; 27(12): 1561-1572.e7
Non Patent Literature 2: Konagaya et al., Scientific Reports. 2019 Jan 24; 9(1): 640

### SUMMARY OF INVENTION

### Technical Problem

Accordingly, it is an object of the present invention to develop a method with which pancreatic endoderm cells, which are cells on a more differentiated stage compared with posterior foregut cells, can be proliferated while they retain the differentiation potential (i.e., expansion culture is possible).

### Solution to Problem

In order to solve the foregoing problems, the inventors of the present invention focused on senescence-related reagents and screened the senescence-related reagents for a reagent that potentially enables expansion culture of pancreatic endoderm cells. As a result, it was found that Y-27632, a ROCK inhibitor, significantly increased the number of pancreatic endoderm cells, and ROCK inhibitors other than Y-27632 also exhibited a similar effect of proliferating pancreatic endoderm cells. When further investigation was conducted based on these findings, it was demonstrated that expansion culture of pancreatic endoderm cells could be conducted by adding Y-27632 to the culture medium such that the concentration was 10 µM or 50 µM.

In cell culture, a low concentration (e.g., 10 µM) of Y-27632 is sometimes used on only the first day of reseeding from the viewpoint of suppressing apoptosis. However, it is known that long-term use of the ROCK inhibitor such as Y-27632 alters the differentiation state and the like of cells (Maldonado M. et al., Stem Cell Res 17; 222-227; 2016), and use of the ROCK inhibitor for more than 1 day has been avoided. Therefore, it was very surprising that, even when a high concentration (10 µM or more) of Y-27632 was used, and Y-27632 was used for a long period of time during culture, efficient expansion culture of pancreatic endoderm cells was possible for a long period of time while the differentiation potential was retained (in an aspect, it was possible to proliferate the cells by a factor of 1×10⁵ or more after 60-day or more culture).

The inventors of the present invention further investigated a mechanism by which the ROCK inhibitor exhibited the effect of proliferating pancreatic endoderm cells. It was suggested that the main mechanism by which such an effect was exhibited was not based on an anti-apoptosis effect, but based on suppression of cellular senescence and the resulting suppression of fibrosis or epithelial-mesenchymal transition. The inventors conducted further studies based on these findings and have achieved the present invention.

That is to say, the present invention is as follows.
[1-1] A method for producing a pancreatic endoderm cell, comprising a step of culturing a pancreatic endoderm cell in a culture medium containing: a ROCK inhibitor; and KGF and/or EGF.
[1-2] The method according to [1-1], wherein the culture medium contains both KGF and EGF.
[1-3] The method according to [1-1] or [1-2], wherein the culture medium contains nicotinamide.
[2] The method according to any one of [1-1] to [1-3], wherein the culture is conducted for 2 days or more.
[3] The method according to any one of [1-1] to [2], wherein the ROCK inhibitor is selected from the group consisting of Y-27632, GSK269962, GSK429286A, Fasudil hydrochloride, H1152, and Thiazovivin.
[4] The method according to any one of [1-1] to [2], wherein the ROCK inhibitor is Y-27632.
[5] The method according to any one of [1-1] to [4], wherein the culture medium contains a TGF-β inhibitor and/or a retinoic acid-receptor agonist.
[6] The method according to [5], wherein the TGF-β inhibitor is 2-[3-[6-methylpyridin-2-yl]-1H-pyrazol-4-yl]-1,5-naphthyridine.
[7] The method according to [5] or [6], wherein the retinoic acid-receptor agonist is retinoic acid.
[8] The method according to any one of [1-1] to [7], wherein the pancreatic endoderm cell is derived from a pluripotent stem cell.
[9] The method according to any one of [1-1] to [8], wherein the pancreatic endoderm cell is a cell derived from a patient with a hereditary pancreatic disease.
[10-1] The method according to any one of [1-1] to [9], wherein the culture is conducted under feeder-free conditions.
[10-2] The method according to any one of [1-1] to [10-1], wherein the culture is conducted under xeno-free conditions.
[11] A pancreatic endoderm cell produced by the method according to any one of [1-1] to [10-2].
[12-1] A kit for expansion culture of a pancreatic endoderm cell, comprising: a ROCK inhibitor; and KGF and/or EGF.
[12-2] The kit according to [12-1], comprising both KGF and EGF.
[12-3] The kit according to [12-1] or [12-2], comprising nicotinamide.
[13-1] The kit according to any one of [12-1] to [12-3], comprising a TGF-β inhibitor and/or a retinoic acid-receptor agonist.
[13-2] The kit according to [13-1], comprising both the TGF-β inhibitor and the retinoic acid-receptor agonist.
[13-3] The kit according to [13-1] or [13-2], wherein the TGF-β inhibitor is 2-[3-[6-methylpyridin-2-yl]-1H-pyrazol-4-yl]-1,5-naphthyridine.
[13-4] The kit according to any one of [13-1] to [13-3], wherein the retinoic acid-receptor agonist is retinoic acid.
[14-1] A method for producing a β-like cell or a progenitor cell thereof, comprising a step of inducing differentiation of the pancreatic endoderm cell according to [11] into a β-like cell or a progenitor cell thereof.
[14-2] A cell produced by the method according to [14-1].
[15] An agent for cell transplantation therapy, comprising the cell according to 1 1] or [14-2].
[16] The agent according to [15] for treating diabetes.
[17-1] A method for treating a pancreatic disease in a mammal, comprising administering an effective amount of the cell according to [11] or [14-2], or the agent according to [15] or [16] to the mammal.
[17-2] The method according to [17-1], wherein the pancreatic disease is diabetes.
[18-1] The cells according to [11] or [14-2], or the agent according to [15] or [16] for use in the treatment of a pancreatic disease.
[18-2] The cell or the agent according to [18-1], wherein the pancreatic disease is diabetes.
[19-1] Use of the cell according to [11] or [14-2], or the agent according to [15] or [16] in the manufacture of a therapeutic medicine for a pancreatic disease.
[19-2] The cell or the agent according to [19-1], wherein the pancreatic disease is diabetes.

### Advantageous Effects of Invention

With the present invention, it is possible to proliferate pancreatic endoderm cells with high efficiency. The thus proliferated cells, and pancreatic endocrine cells such as pancreatic β cells, and pancreatic exocrine cells that are obtained by inducing differentiation of the proliferated cells above are also important for clinical use. Also, these cells are useful in regenerative medicine, drug discovery screening, and the like for pancreatic diseases instead of human pancreatic islets, exocrine cells, and tissues, which are not easily available as research specimens.

### Brief Description of Drawings

[FIG. 1] A diagram illustrating the outline of a stepwise differentiation-inducing method for β-like cells.
[FIG. 2-A] A schematic diagram illustrating senescence-related reagent screening. NKX6.1-positive pancreatic endoderm cells were seeded on a 24-well plate and various senescence-related reagents were added to a conventional stage-4 culture medium to conduct the screening. After the elapse of one week, the cells were counted under a fluorescence microscope.
[FIG. 2-B] The number of NKX6.1-positive cells in culture with various senescence-related reagents. Number of replicate experiments = 3; number of cells = mean of results from three replicates; using Dunnett's test. Error bars indicate standard errors.
[FIG. 2-C] The fraction of NKX6.1-positive cells in culture with various senescence-related reagents. Number of replicate experiments = 3; fraction = mean fraction of results from three replicates; using Dunnett's test. Error bars indicate standard errors.
[FIG. 2-D] The fraction of β gal-positive cells generated by various senescence-related reagents. Number of replicate experiments = 3; fraction = mean fraction of results from three replicates; using Dunnett's test. Error bars indicate standard errors.
[FIG. 3-A] A schematic diagram illustrating an experiment of examining other ROCK inhibitors. NKX6.1-positive pancreatic endoderm cells were seeded on a 24-well plate and various ROCK inhibitors were added to a conventional stage-4 culture medium to conduct the experiment. After the elapse of one week, the cells were counted under a fluorescence microscope.
[FIG. 3-B] The total number of cells in culture with various ROCK inhibitors. Number of replicate experiments = 3; using one-way analysis of variance (One-way ANOVA). Error bars indicate standard errors.
[FIG. 3-C] The number of NKX6.1-positive cells and the fraction of NKX6.1-positive cells in culture with various ROCK inhibitors. Number of replicate experiments = 3; using one-way analysis of variance. Error bars indicate standard errors.
[FIG. 4-A] A schematic diagram illustrating expansion culture of pancreatic endoderm cells with Y-27632 (50 µM). NKX6.1-positive pancreatic endoderm cells were seeded on a plate and cultured in a culture medium containing Y-27632 (50 µM) for one week. After the elapse of one week, the cells were reseeded, and passage and expansion culture were repeated.
[FIG. 4-B] A change in the total number of cells during expansion culture with Y-27632 (50 µM). n = 3.
[FIG. 4-C] A change in the number of pancreatic endoderm cells during expansion culture with Y-27632 (50 µM). n = 3.
[FIG. 4-D] Immunostained images of pancreatic endoderm cells after expansion culture with Y-27632 (50 µM).
[FIG. 4-E] Flow cytometry scatter plots illustrating NKX6.1 expression and Ki67 expression in cells after expansion culture with Y-27632 (50 µM).
[FIG. 4-F] A change in the Ki67-positive ratio during expansion culture with Y-27632 (50 µM). Number of replicate experiments = 3; error bars indicate standard errors.
[FIG. 4-G] An immunostained image of a pancreatic islet-like cell cluster obtained by inducing differentiation of pancreatic endoderm cells after expansion culture had been conducted twice.
[FIG. 4-H] Changes in the number of pancreatic endoderm cells derived from ES cells (KhES-3 line) during expansion culture.
[FIG. 5-A] Changes in the number of PDX1⁺/NKX6.1⁺ pancreatic endoderm cells and the total number of cells during expansion culture with Y-27632 (0, 10, or 50 µM).
[FIG. 5-B] Immunostained images of pancreatic endoderm cells derived from the KhES-3 line after passaged and cultured 5 times (5 weeks) with Y-27632 (10 or 50 µM).
[FIG. 5-C] Results of quantitative evaluation of the nuclear morphology of all the cells in NKX6.1 immunostaining in FIG. 5-B (n = 616 cells (10 µM Y-27632); n = 519 cells (50 µM Y-27632); biological replicate = 1). The eccentricity (circularity) was compared between the both groups using the Mann-Whitney U test.
[FIG. 6] Images of pancreatic endoderm cells immunostained against α-SMA at the third passage of the pancreatic endoderm cells with or without the administration of Y-27632 (50 µM).
[FIG. 7] The fraction of NKX6.1-positive cells in culture with various screening reagents.
[FIG. 8] Changes in the cumulative number of NKX6.1⁺ pancreatic endoderm cells during expansion culture using a DMSO group (Y-27632 + DMSO) or an ALK5 inhibitor (ALK5i) + retinoic acid (RA) group (Y-27632 + ALK5i + RA). Number of replicate experiments = 4.
[FIG. 9] The ratio of the number of β-like cells obtained by inducing differentiation of pancreatic endoderm cells after expansion culture had been conducted 5 times using the DMSO group (Y-27632 + DMSO) or the ALK5i + RA group (Y-27632 + ALK5i + RA). The results were compared with that from a control group in which pancreatic endoderm cells that had not been passaged (P0) were induced to β-like cells. Number of replicate experiments = 3; error bars indicate standard errors.

### DESCRIPTION OF EMBODIMENTS

### 1. Method for Producing Pancreatic Endoderm Cells

The present invention provides a method for producing pancreatic endoderm cells using a culture medium containing a ROCK inhibitor. Specifically, the present invention provides a method for producing pancreatic endoderm cells (which may also be referred to as a "production method of the present invention" hereinafter) that includes a step of culturing pancreatic endoderm cells in a culture medium containing a ROCK inhibitor and KGF and/or EGF. The production method of the present invention may also include a step of isolating produced pancreatic endoderm cells.

With the production method of the present invention, pancreatic endoderm cells self-proliferate, and thus the same type of cells are produced. That is to say, the production method of the present invention can also be referred to as a "method for proliferating pancreatic endoderm cells or a method of expansion culture of pancreatic endoderm cells that includes a step of culturing pancreatic endoderm cells in a culture medium containing: a ROCK inhibitor; and KGF and/or EGF".

In this specification, the term "pancreatic endoderm cells" means cells that have at least a potential to differentiate into β-like cells and express at least PDX1 and NKX6.1. The pancreatic endoderm cells may be cells that further express one or more gene markers such as SOX9 and GATA4. Also, in this specification, the term "β-like cells" means cells that are obtained by inducing differentiation of endocrine progenitor cells or immature β cells in vitro, have the same characteristics as, or similar characteristics to, those of in-vivo pancreatic β cells, and express and/or secret insulin.

In this specification, the term "cells" encompasses a "cell population" unless otherwise stated. The cell population may be composed of one type of cell, or may be composed of two or more types of cells.

In this specification, the term "expansion culture" means culture conducted for the purpose of increasing the number of cells by proliferating a desired cell population. It is sufficient that the increase in the number of cells is achieved by the number of cells increased through cell proliferation being larger than the number of cells reduced through cell death, and it is not necessary that all the cells in the cell population are proliferated. The number of cells may increase by a factor of 1.1, 1.2, 1.5, 2, 3, 4, 5, 6, 7, 8, 9, 10, 15, 20, 30, 40, 50, 100, 300, 500, 1000, 3000, 5000, 10000, 100000, or 1000000 or more compared with that before expansion culture.

A basal culture medium used in the production method of the present invention is not particularly limited, but examples thereof include StemFit (registered trademark) AK02 culture medium (Ajinomoto Co., Inc.), StemFit (registered trademark) AK03 culture medium (Ajinomoto Co., Inc.), StemFit (registered trademark) Basic03 culture medium, CTS (registered trademark) KnockOut SR XenoFree Medium (Gibco), mTeSR1 culture medium, TeSR1 culture medium, (Stem Cell Technologies), Iscove's modified Dulbecco's medium (GE HealthCare Technologies Inc.), improved MEM (Thermo Fisher Scientific Inc.), and the like. Among these, the improved MEM culture medium is preferable. These culture media can also be used for culture under feeder-free and xeno-free conditions. Examples of other basal culture media include RPMI-1640 culture medium, EagleMEM (EMEM), Dulbecco's Modified MEM, Glasgow's MEM (GMEM), α-MEM, 199 culture medium, IMDM, DMEM, Hybridoma Serum free culture medium, KnockOut (trademark) DMEM, Advanced TM culture medium (e.g., Advanced MEM, Advanced RPMI, and Advanced DMEM/F-12), Ham's Medium F-12, Ham's Medium F-10, Ham's Medium F12K, DMEM/F-12, ATCC-CRCM30, DM-160, DM-201, BME, Fischer, McCoy's 5A, Leibovitz's L-15, RITC80-7, MCDB105, MCDB107, MCDB131, MCDB153, MCDB201, NCTC109, NCTC135, Waymouth's Medium (e.g., Waymouth's MB752/1), CMRL culture medium (e.g., CMRL-1066), Williams' medium E, Brinster's BMOC-3 Medium, E8 Medium, StemPro 34, and MesenPRO RS (these are available from Thermo Fisher Scientific Inc.); ReproFF2, Primate ES Cell Medium, and ReproStem (these are available from REPROCELL Inc.); ProculAD (available from ROHTO Pharmaceutical Co., Ltd.); MSCBM-CD and MSCGM-CD (these are available from Lonza); EX-CELL302 culture medium (available from SAFC) or EX-CELL-CD-CHO (available from SAFC); ReproMed (trademark) iPSC Medium (available from REPROCELL Inc.); mixtures thereof; and the like, but there is no limitation thereto.

As shown in the section "Examples" below, it was observed that various types of ROCK inhibitors had an effect of proliferating pancreatic endoderm cells. That is to say, it is important to suppress the functions of a Rho-kinase (ROCK) to proliferate pancreatic endoderm cells, and any ROCK inhibitor can be applied to the production method of the present invention as long as it can suppress the functions. Examples of the ROCK inhibitor include Y-27632 (see, for example, Ishizaki et al., Mol. Pharmacol. 57, 976-983 (2000); and Narumiya et al., Methods Enzymol. 325, 273-284 (2000)), Fasudil/HA1077 (see, for example, Uenata et al., Nature 389: 990-994 (1997)), SR3677 (see, for example, Feng Y et al., J Med Chem. 51: 6642-6645 (2008)), GSK269962 (see, for example, Stavenger RA et al., J Med Chem. 50: 2-5 (2007) or WO2005/037197), GSK429286A, H1152 (see, for example, Sasaki et al., Pharmacol. Ther. 93: 225-232 (2002)), Wf-536 (see, for example, Nakajima et al., Cancer Chemother Pharmacol. 52(4): 319-324 (2003)), Thiazovivin, salts or derivatives thereof, and the like. Also, examples of other ROCK inhibitors include an antisense nucleic acid against ROCK, an RNA interference inducible nucleic acid (e.g., siRNA), a dominant-negative mutant, expression vectors therefor, and the like. Other known low-molecular-weight compounds and salts or derivatives thereof can also be used as the ROCK inhibitor (see, for example, U.S. Publication No. 2005/0209261, U.S. Publication No. 2005/0192304, U.S. Publication No. 2004/0014755, U.S. Publication No. 2004/0002508, U.S. Publication No. 2004/0002507, U.S. Publication No. 2003/0125344, U.S. Publication No. 2003/0087919, WO 2003/062227, WO 2003/059913, WO 2003/062225, WO 2002/076976, and WO 2004/039796). Among these ROCK inhibitors, Y-27632, GSK269962, GSK429286A, Fasudil hydrochloride, H1152, and Thiazovivin are preferable, and Y-27632 is particularly preferable. In the production method of the present invention, only one of the ROCK inhibitors may be used, or two or more of them may be used.

Examples of a salt of a compound include base addition salts such as inorganic base salts (e.g., alkali metal salts (sodium salts, potassium salts, and the like), alkaline earth metal salts (calcium salts, magnesium salts, and the like), aluminum salts, and ammonium salts) and organic base salts (e.g., trimethylamine, triethylamine, pyridine, picoline, ethanolamine, diethanolamine, triethanolamine, dicyclohexylamine, and N,N'-dibenzylethylenediamine); and acid addition salts such as inorganic acid salts (e.g., hydrochlorides, hydrobromides, sulfates, hydroiodides, nitrates, and phosphates) and organic acid salts (e.g., citrates, oxalates, acetates, formates, propionates, benzoates, trifluoroacetates, maleates, tartrates, methanesulfonates, benzenesulfonates, and p-toluenesulfonates).

When Y-27632 or a salt thereof (e.g., Y-27632 dihydrochloride) is used as the ROCK inhibitor, the concentration in the culture medium is typically 1 µM or more (e.g., 5 µM, 10 µM, 15 µM, 20 µM, 25 µM, 30 µM, 35 µM, 40 µM, 45 µM, 50 µM, or higher concentration) and 500 µM or less (e.g., 400 µM, 300 µM, 200 µM, 150 µM, 100 µM, or lower concentration). The concentration of Y-27632 in the culture medium may be 0.1 µM to 1000 µM, 1 µM to 300 µM, or 10 µM to 100 µM. In an aspect, the concentration is 50 µM.

When GSK269962 or a salt thereof (e.g., GSK269962 hydrochloride) is used as the ROCK inhibitor, the concentration in the culture medium is typically 0.02 µM to 100 µM, preferably 0.2 µM to 50 µM, and more preferably 2 µM to 10 µM. In an aspect, the concentration is 2 µM.

When GSK429286A or a salt thereof is used as the ROCK inhibitor, the concentration in the culture medium is typically 0.02 µM to 100 µM, preferably 0.2 µM to 50 µM, and more preferably 2 µM to 10 µM. In an aspect, the concentration is 2 µM.

When Fasudil or a salt thereof (e.g., Fasudil hydrochloride) is used as the ROCK inhibitor, the concentration in the culture medium is typically 0.1 µM to 500 µM, preferably 1 µM to 200 µM, and more preferably 10 µM to 50 µM. In an aspect, the concentration is 50 µM.

When H1152 or a salt thereof (e.g., H1152 dihydrochloride) is used as the ROCK inhibitor, the concentration in the culture medium is typically 0.02 µM to 100 µM, preferably 0.2 µM to 50 µM, and more preferably 2 µM to 10 µM. In an aspect, the concentration is 2 µM.

When Thiazovivin or a salt thereof is used as the ROCK inhibitor, the concentration in the culture medium is typically 0.02 µM to 100 µM, preferably 0.2 µM to 50 µM, and more preferably 2 µM to 10 µM. In an aspect, the concentration is 2 µM or 10 µM.

KGF is a protein called a keratinocyte growth factor and may also be called FGF-7. KGF that is commercially available from, for example, R&D Systems, Inc. or the like can be used. The concentration of KGF in the culture medium is typically 1 ng/ml to 1 µg/ml, preferably 5 ng/ml to 500 ng/ml, and more preferably 10 ng/ml to 200 ng/ml (e.g., 100 ng/ml).

EGF is a protein called an epithelial growth factor or an epidermal growth factor. EGF that is commercially available from, for example, R&D Systems, Inc. or the like can be used. The concentration of EGF in the culture medium is typically 1 ng/ml to 1 µg/ml, preferably 5 ng/ml to 500 ng/ml, and more preferably 10 ng/ml to 100 ng/ml (e.g., 50 ng/ml).

The culture medium may contain nicotinamide. The concentration of nicotinamide in the culture medium is typically 0.1 mM to 200 mM, preferably 1 mM to 100 mM, and more preferably 5 mM to 50 mM (e.g., 10 mM).

As necessary, the culture medium may include an additive for a culture medium other than those listed above, such as serum (e.g., fetal bovine serum (FBS), horse serum, or the like) or one or more serum alternatives (e.g., Knockout Serum Replacement (KSR), N2 Supplement (Invitrogen), B27 Supplement (Invitrogen), albumin, transferrin, apotransferrin, a fatty acid, insulin, a collagen precursor, a trace element, 2-mercaptoethanol, and 3'-thiolglycerol), and moreover, it may also contain one or more substances selected from a lipid, an amino acid, L-glutamine, Glutamax (Invitrogen), a nonessential amino acid, a vitamin, a growth factor, a low-molecular-weight compound, an antibiotic, an antioxidant, pyruvic acid, a buffer, an inorganic salt, selenic acid, progesterone, putrescine, and the like.

In the production method of the present invention, the culture may be conducted under feeder-free conditions and/or xeno-free conditions over the entire period of the culture or during a part of the period of the culture. From the viewpoint of a clinical application, it is preferable to conduct the production method of the present invention under feeder-free conditions and xeno-free conditions throughout the entire period. As used herein, the term "feeder-free" refers to a medium or culturing conditions free of auxiliary cells (i.e., feeder cells) that are different in type from cells to be cultured and used for adjusting culturing conditions for the cells to be cultured. The term "xeno-free" refers to a medium or culturing conditions free of components derived from organisms of biological species that are different from that of cells to be cultured.

In the production method of the present invention, the culture may be suspension culture or adherent culture as long as desired cells can be proliferated, but adherent culture is preferable. In this specification, the term "suspension culture" means culture conducted under conditions that allow cells or cell aggregates to be kept suspended in a culture medium, that is, culture conducted under conditions that prevent strong cell-substratum junctions from being formed between cells or cell aggregates and a culture vessel. In this specification, the term "adherent culture" means culture conducted under conditions that allow strong cell-substratum junctions to be formed between cells or cell aggregates and a culture device or the like.

Examples of a culture vessel to be used for adherent culture include culture vessels whose surfaces have been subjected to artificial treatment (e.g., coating treatment with an extracellular matrix such as a basal membrane preparation, fibronectin, laminin or a fragment thereof, entactin, collagen, gelatin, Synthemax, or vitronectin, or a macromolecule such as polylysine or polyornithine, or surface processing such as positive charge treatment) for the purpose of improving adhesion to cells. Among these, a culture vessel coated with laminin or a fragment thereof is preferable.

Examples of the laminin or a fragment thereof used in the present invention include laminin-111 or a fragment thereof that includes the E8 region, laminin-211 or a fragment thereof that includes the E8 region (e.g., iMatrix-211), laminin-121 or a fragment thereof that includes the E8 region, laminin-221 or a fragment thereof that includes the E8 region, laminin-332 or a fragment thereof that includes the E8 region, laminin-3A11 or a fragment thereof that includes the E8 region, laminin-411 or a fragment thereof that includes the E8 region (e.g., iMatrix-411), laminin-421 or a fragment thereof that includes the E8 region, laminin-511 or a fragment thereof that includes the E8 region (e.g., iMatrix-511 or iMatrix-511 silk), laminin-521 or a fragment thereof that includes the E8 region, laminin-213 or a fragment thereof that includes the E8 region, laminin-423 or a fragment thereof that includes the E8 region, laminin-523 or a fragment thereof that includes the E8 region, laminin-212/222 or a fragment thereof that includes the E8 region, laminin-522 or a fragment thereof that includes the E8 region, and the like.

The culture vessel to be used for suspension culture is not particularly limited as long as "suspension culture" can be conducted using it, and a person skilled in the art could select one as appropriate. Examples of such a culture vessel include a flask, a tissue culture flask, a dish, a Petri dish, a tissue culture dish, a multi-dish, a microplate, a microwell plate, a micropore, a multi-plate, a multi-well plate, a chamber slide, a Schale, a tube, a tray, a culture bag, a roller bottle, and the like. Furthermore, a bioreactor is an example of a vessel for suspension culture. These culture vessels are preferably non-cell-adhesive in order to enable suspension culture. Examples of the non-cell-adhesive culture vessels include culture vessels whose surfaces have not been subjected to artificial treatment (e.g., coating treatment with an extracellular matrix or the like) for improving adhesion to cells.

The culture temperature is not particularly limited, but is about 30°C to 40°C and preferably about 37°C. The culture is conducted under a CO₂-containing air atmosphere, and the CO₂ concentration is preferably about 2% to 5%.

In the production method of the present invention, target cells can be obtained for a long period of time, and therefore, the culture period is not particularly limited, but the culture is typically conducted for 2 days or more (e.g., 3 days, 4 days, 5 days, 6 days, 7 days, 8 days, 9 days, 10 days, 20 days, or more days) and 100 days or less (e.g., 90 days, 80 days, 70 days, 60 days, or less days). In an aspect, the culture period may be more than 100 days (e.g., 110 days, 120 days, or more days) because pancreatic endoderm cells can also be proliferated for more than 100 days using the production method of the present invention.

The culture density of cells is not particularly limited as long as the cells can be proliferated. The culture density is typically 1.0×10² to 1.0×10⁷ cells/cm², preferably 1.0×10³ to 1.0×10⁶ cells/cm², and more preferably 1.0×10⁴ to 1.0×10⁵ cells/cm².

Pancreatic endoderm cells used in the production method of the present invention may be those isolated from a living body or those obtained from a commercially available cell line, but are preferably those derived from pluripotent stem cells. In an aspect, pancreatic endoderm cells used as the starting cells in the production method of the present invention are derived from a patient with a hereditary pancreatic disease. Examples of the pancreatic disease include acute pancreatitis, chronic pancreatitis, type 1 diabetes, type 2 diabetes, pancreatic tumor, tumor of the Langerhans' islands, and the like. Among pancreatic diseases, a hereditary pancreatic disease is caused by a genetic abnormality. Specific examples of the hereditary pancreatic disease include hereditary pancreatitis, familial pancreatic tumor, cystic fibrosis, and the like, but are not limited thereto. The pancreatic endoderm cells derived from a patient with a hereditary pancreatic disease may be those isolated from the patient, but are preferably pancreatic endoderm cells produced by initializing somatic cells derived from the patient, establishing iPS cells from the initialized somatic cells, and inducing differentiation of the iPS cells using a method known per se. Pancreatic endoderm cells derived from a patient with a hereditary pancreatic disease, and cells such as β-like cells obtained by inducing differentiation of the pancreatic endoderm cells can be used as a pancreatic disease model that reflects the state of the disease, and are thus suitable for, for example, screening of a drug for treating or preventing the pancreatic disease.

As used herein, the term "pluripotent stem cells" refers to stem cells that can differentiate into tissue or cells having various different forms or functions in the body, and that can also differentiate into cells of any lineages of the three germ layers (endoderm, mesoderm and ectoderm). Examples of pluripotent stem cells to be used for the present invention include induced pluripotent stem cells (iPS cells), embryonic stem cells (ES cells), embryonic stem cells from cloned embryos obtained by nuclear transfer (nuclear transfer Embryonic stem cells; ntES cells), multipotent germline stem cells (mGS cells) and embryonic germ cells (EG cells), of which iPS cells (and especially human iPS cells) are preferred. When the pluripotent stem cells are ES cells or arbitrary cells derived from human embryos, the cells may be obtained by destruction of the embryo or they may be obtained without destruction of the embryo, but preferably they are cells obtained without destruction of the embryo.

ES cells are stem cells having pluripotency and auto-replicating proliferation potency, established from the inner cell mass of an early embryo (such as the blastocyst) of a mammal such as a human or mouse. ES cells were discovered in mice in 1981 (M.J. Evans and M.H. Kaufman (1981), Nature 292:154-156), and ES cell lines were later established in primates including humans and monkeys (J.A. Thomson et al. (1998), Science 282:1145-1147; J.A. Thomson et al. (1995), Proc. Natl. Acad. Sci. USA, 92:7844-7848; J.A. Thomson et al. (1996), Biol. Reprod., 55:254-259; J.A. Thomson and V.S. Marshall (1998), Curr. Top. Dev. Biol., 38:133-165). ES cells can be established by extracting the inner cell mass from the blastocyst of a fertilized egg of a target animal and culturing the inner cell mass on a fibroblast feeder. Alternatively, ES cells can be established using a single embryo blastomere alone during the cleavage stage before the blastocyst stage (Chung Y. et al. (2008), Cell Stem Cell 2: 113-117), or they can be established using a developmentally arrested embryo (Zhang X. et al. (2006), Stem Cells 24: 2669-2676.).

ntES cells are ES cells derived from a clone embryo prepared by nuclear transfer, and they have approximately the same properties as fertilized egg-derived ES cells (Wakayama T. et al. (2001), Science, 292:740-743; S. Wakayama et al. (2005), Biol. Reprod., 72:932-936; Byrne J. et al. (2007), Nature, 450:497-502). In other words, ntES (nuclear transfer ES) cells are ES cells established from the inner cell mass of a cloned embryo-derived blastocyst obtained by exchanging an unfertilized egg nucleus with a somatic cell nucleus. Combinations of nuclear transfer (Cibelli J.B. et al. (1998), Nature Biotechnol., 16:642-646) and ES cell preparation techniques (described above) are used to prepare ntES cells (Wakayama, S. (2008), Jikken Igaku, Vol.26, No.5 (special edition), pp.47-52). For nuclear transfer, a somatic cell nucleus may be implanted into a mammalian enucleated unfertilized egg and cultured for several hours for reprogramming.

The ES cell line used for the present invention may be mouse ES cells, and for example, the different mouse ES cell lines established by the inGenious Targeting Laboratory, Riken (Riken Research Institute), etc., may be used, or for human ES cell lines, the different human ES cell lines established by the University of Wisconsin, NIH, Riken, Kyoto University, the National Center for Child Health and Development, Cellartis, etc., for example, may be used. Specific examples of human ES cell lines include CHB-1 to CHB-12 lines, RUES1 line, RUES2 line and HUES1 to HUES28 lines distributed by ESI Bio Co., H1 and H9 lines distributed by WiCell Research, KhES-1, KhES-2, KhES-3, KhES-4, KhES-5, SSES1, SSES2 and SSES3 distributed by Riken, and so on.

iPS cells are cells obtained by reprogramming of mammalian somatic cells or undifferentiated stem cells by introduction of specific factors (nuclear reprogramming factors). A large number of iPS cells currently exist, among which there may be used human iPSCs established by introduction of the 4 factors Oct3/4·Sox2·Klf4·c-Myc into human fibroblasts by Yamanaka (Takahashi K, Yamanaka S., et al. Cell, (2007) 131:861-872.), Nanog-iPSCs established by selecting of Nanog expression markers after introduction of the 4 factors (Okita, K., Ichisaka, T. and Yamanaka, S. (2007). Nature 448, 313-317.), iPSCs produced by methods without c-Myc (Nakagawa M, Yamanaka S., et al. Nature Biotechnology, (2008) 26, 101-106), iPSCs established by introduction of 6 factors by a virus-free method (Okita K et al. Nat. Methods 2011 May; 8(5):409-12, Okita K et al. Stem Cells. 31(3):458-66.), and so on. The induced pluripotent stem cells established by introduction of the 4 factors OCT3/4·SOX2·NANOG·LIN28, created by Thomson et al. (Yu J., Thomson JA. et al., Science (2007) 318: 1917-1920), the induced pluripotent stem cells created by Daley et al. (Park IH, Daley GQ. et al., Nature (2007) 451:141-146) and the induced pluripotent stem cells created by Sakurata et al. (Japanese Unexamined Patent Publication No. 2008-307007), etc., may also be used.

In addition, any induced pluripotent stem cells publicly known in the field as described in published journal (for example, Shi Y, Ding S., et al., Cell Stem Cell, (2008) Vol.3, Issue 5, 568-574, Kim JB., Scholer HR., et al., Nature, (2008) 454, 646-650; Huangfu D., Melton, DA., et al., Nature Biotechnology, (2008) 26, No 7, 795-797), or patent publications (for example, Japanese Unexamined Patent Publication No. 2008-307007, Japanese Unexamined Patent Publication No. 2008-283972, US2008-2336610, US2009-047263, WO2007-069666, WO2008-118220, WO2008-124133, WO2008-151058, WO2009-006930, WO2009-006997 and WO2009-007852), may also be used.

Induced pluripotent stem cell lines that are iPSC lines established by NIH, Riken, Kyoto University, etc., for example, may be used as well. Examples include human iPSC lines such as HiPS-RIKEN-1A line, HiPS-RIKEN-2A line, HiPS-RIKEN-12A line, Nips-B2 line, etc., by Riken, 253G1 line, 253G4 line, 1201C1 line, 1205D1 line, 1210B2 line, 1383D2 line, 1383D6 line, 201B7 line, 409B2 line, 454E2 line, 585A1 line, 606A1 line, 610B1 line, 648A1 line, 1231A3 line, FfI-01s04 line, etc., by Kyoto University, with the 585A1 line being preferred.

mGS cells are pluripotent stem cells derived from the testis and are the source cells for spermatogenesis. Like ES cells, these cells can be induced to differentiate into cells of various lineages, and have properties such as the ability to produce chimeric mice when transplanted into mouse blastocysts (Kanatsu-Shinohara M. et al. (2003) Biol. Reprod., 69:612-616; Shinohara K. et al. (2004), Cell, 119:1001-1012). These cells are capable of auto-replication in a culture medium containing glial cell line-derived neurotrophic factor (GDNF), and by repeated passage under the same culture conditions as those for ES cells, germline stem cells can be obtained (Takebayashi, M. et al. (2008), Jikken Igaku, Vol.26, No.5 (special edition), pp.41-46, Yodosha Co., Ltd. (Tokyo, Japan)).

EG cells are established from the primordial germ cells in the embryonic period and have pluripotency similar to that of ES cells. They can be established by culturing primordial germ cells in the presence of substances such as LIF, bFGF, and stem cell factor (Matsui Y. et al. (1992), Cell, 70:841-847; J. L. Resnick et al. (1992), Nature, 359:550-551).

The source species of the pluripotent stem cells is not particularly restricted, and for example, the cells may be from a rodent such as a rat, mouse, hamster or guinea pig, a lagomorph such as a rabbit, an ungulate such as a pig, cow, goat or sheep, a carnivora such as a dog or a cat, or a primate such as a human, monkey, rhesus monkey, marmoset, orangutan or chimpanzee. The preferred source species is human.

Differentiation of pluripotent stem cells into pancreatic endoderm cells can be induced using known methods such as methods described in Toyoda T, et al. Stem Cell Reports 2017, Patent Literature 1, Non Patent Literature 1, Non Patent Literature 2, and WO 2017/047797. Specifically, for example, the differentiation can be induced using a method that includes:
A) a step of inducing differentiation of pluripotent stem cells into embryonic endoderm cells;
B) a step of inducing differentiation of the embryonic endoderm cells into primitive gut tube cells;
C) a step of inducing differentiation of the primitive gut tube cells into posterior foregut cells; and
D) a step of inducing differentiation of the posterior foregut cells into pancreatic endoderm cells,
and the like.

A basal culture medium and additives for a culture medium used in the steps A) to D) may be the same as the basal culture medium and the additives for a culture medium used in the production method of the present invention. In the steps A) to D), the culture temperature is typically about 30°C to 40°C and preferably about 37°C. The culture is conducted under a CO₂-containing air atmosphere, and the CO₂ concentration is preferably about 2% to 5%. In the steps A) to D), it is preferable to conduct adherent culture. The definition and way of the adherent culture are as described above.

In the step A), the differentiation into embryonic endoderm cells can be conducted by, for example, culturing pluripotent stem cells in a culture medium containing a low dose of activin A. The culture medium may contain a ROCK inhibitor and a GSK3β inhibitor. The culture period is typically 2 days to 8 days.

The concentration of activin A used in the step A) in the culture medium is, for example, 5 to 1000 ng/mL, preferably 20 to 500 ng/mL, and more preferably 50 to 150 ng/mL.

Examples of the GSK3β inhibitor used in the step A) include CHIR98014, CHIR99021, TDZD-8, SB216763, TWS-119, Kenpaullone, 1-Azakenpaullone, SB216763, SB415286, AR-AO144-18, CT99021, CT20026, and the like. Among these, CHIR99021 is preferable. When CHIR99021 is used, the concentration in the culture medium is typically 0.5 to 5 µM and preferably 1 to 4 µM.

The ROCK inhibitor used in the step A) may be the same as the ROCK inhibitor used in the production method of the present invention. When Y-27632 is used as the ROCK inhibitor, the concentration in the culture medium is typically 1 to 20 µM and preferably 5 to 15 µM.

Insulin can also be added to the culture medium. The concentration of insulin in the culture medium is typically 0.01 to 20 µM, preferably 0.1 to 10 µM, and more preferably 0.5 to 5 µM. The concentration of insulin in the culture medium may be the concentration of insulin included in the B-27 supplement added, but is not limited thereto.

In the step B), the differentiation into primitive gut tube cells can be conducted by, for example, culturing the embryonic endoderm cells obtained in the step A) in a culture medium containing a growth factor. The culture period is typically 2 days to 8 days.

The growth factor used in the step B) is preferably EGF, KGF, and FGF10, more preferably EGF and/or KGF, and even more preferably KGF. The concentration of the growth factor in the culture medium is determined as appropriate depending on the type of growth factor used, but is typically about 0.1 nM to 1000 µM and preferably about 0.1 nM to 100 µM. When EGF is used, the concentration is about 5 to 2000 ng/ml (i.e., about 0.8 to 320 nM), preferably about 5 to 1000 ng/ml (i.e., about 0.8 to 160 nM), and more preferably about 10 to 1000 ng/ml (i.e., about 1.6 to 160 nM). When FGF10 is used, the concentration is about 5 to 2000 ng/ml (i.e., about 0.3 to 116 nM), preferably about 10 to 1000 ng/ml (i.e., about 0.6 to 58 nM), and more preferably about 10 to 1000 ng/ml (i.e., about 0.6 to 58 nM). For example, when KGF is used as the growth factor, the concentration is typically 5 to 150 ng/mL, preferably 30 to 100 ng/mL, and particularly preferably about 50 ng/mL.

In the step C), the differentiation into posterior foregut cells can be conducted by, for example, culturing the primitive gut tube cells obtained in the step B) in a culture medium containing a growth factor, a retinoic acid-receptor agonist such as a retinoic acid derivative, a Hedgehog signal inhibitor, and a BMP inhibitor. The culture period is typically 1 day to 5 days.

The content described in the step B) is applied to the type and concentration of the growth factor used in the step C).

Examples of the retinoic acid-receptor agonist used in the step C) include retinoic acid (all-trans-3,7-dimethyl-9-(2,6,6-trimethyl-1-cyclohexen-1-yl)-2,4,6,8-nonatetraenoic acid (Cas No: 302-79-4)), retinoates, retinoic acid precursors, retinoic acid derivatives, and the like. Examples of the retinoates include sodium retinoate, potassium retinoate, calcium retinoate, and the like. Examples of the retinoic acid precursors include β-carotene, retinol esters, retinol, retinal, and the like. The term "retinoic acid derivative" means artificially modified retinoic acid retaining the functions of natural retinoic acid, and examples thereof include 4-[[(5,6,7,8-tetrahydro-5,5,8,8-tetramethyl-2-naphthalenyl)carbonyl]amino]-benzoic acid (AM580) (Tamura K, et al., Cell Differ. Dev. 32: 17-26 (1990)), 4-[(1E)-2-(5,6,7,8-tetrahydro-5,5,8,8-tetramethyl-2-naphthalenyl)-1-propen-1-yl]-benzoic acid (TTNPB) (Strickland S, et al., Cancer Res. 43: 5268-5272 (1983)), retinol palmitate, retinol, retinal, 3-dehydroretinoic acid, 3-dehydroretinol, 3-dehydroretinal, and the like. Among these, TTNPB is preferable. When TTNPB is used, the concentration in the culture medium is typically 1 to 50 nM and preferably 5 to 15 nM.

Examples of the Hedgehog pathway inhibitor used in the step C) include cyclopamine, jervine, 3-keto-N-(aminoethyl-aminocaproyl-dihydro-cinnamoyl) (KAAD)-cyclopamine, CUR-61414, SANT-1, SANT-2, SANT-3, SANT-4, IPI-926, IPI-269609, GDC-0449, NVP-LDE-225, and the like. Among these, SANT-1 is preferable. When SANT-1 is used, the concentration in the culture medium is typically 100 to 500 nM and preferably 50 to 150 nM.

Examples of the BMP inhibitor used in the step C) include proteinous inhibitors such as chordin, noggin, and follistatin, dorsomorphin (i.e., 6-[4-(2-piperidin-1-yl-ethoxy)phenyl]-3-pyridin-4-yl-pyrazolo[1,5-a]pyrimidine), dorsomorphin derivatives (P. B. Yu et al. (2007), Circulation, 116:II_60; P.B. Yu et al. (2008), Nat. Chem. Biol., 4:33-41; J. Hao et al. (2008), PLoS ONE, 3(8):e2904), LDN-193189, and the like. Among these, LDN-193189 is preferable. When LDN-193189 is used, the concentration in the culture medium is typically 10 to 1000 nM and preferably 100 to 300 nM.

In the step D), the differentiation into pancreatic endoderm cells can be conducted by, for example, culturing posterior foregut cells obtained in the step C) in a culture medium containing a growth factor and a BMP inhibitor. The culture medium may contain a nonmuscle myosin II inhibitor, a TGF-β inhibitor, nicotinamide, and the like. The culture period is typically 2 days to 10 days.

When the step D) is conducted, the posterior foregut cells obtained in the step C) may be subjected to 0.25% trypsin-EDTA treatment, dispersed through pipetting, and suspended after centrifugal separation of 0.25% trypsin-EDTA in accordance with a previous report (Toyoda et al., Stem Cell Research (2015) 14, 185-197).

The contents described in the step B) and the step C) are applied to the types and concentrations of the growth factor and the BMP inhibitor used in the step D).

Examples of the nonmuscle myosin II inhibitor used in the step D) include Blebbistatin A3, Calphostin C, Goe6976, Goe7874, Fasudil/HA1077, Hypericin, K-252a, KT5823, ML-7, ML-9, Piceatannol, Staurosporine, W-5, W-7, W-12, W-13, Wortmannin, and the like. Among these, Blebbistatin is preferable. When Blebbistatin is used as the nonmuscle myosin II inhibitor, the concentration in the culture medium is 1 µM to 200 µM and preferably 10 µM to 100 µM.

The TGF-β inhibitor used in the step D) is not particularly limited as long as it is a substance that inhibits the signaling from a TGF-β-bound receptor to SMAD, which means that the substance inhibits binding of TGF-β to the ALK family, the receptor, or phosphorylation of SMAD by the ALK family, and examples thereof include Lefty-1, SB431542, SB202190, SB505124, NPC30345, SD093, SD908, SD208 (Scios), LY2109761, LY364947, LY580276, A-83-01 (WO 2009146408), an ALK5 inhibitor II (2-[3-[6-methylpyridin-2-yl]-1H-pyrazol-4-yl]-1,5-naphthyridine; CAS: 446859-33-2), a TGF-β RI kinase inhibitor VIII (6-[2-tert-butyl-5-[6-methyl-pyridin-2-yl]-1H-imidazol-4-yl]-quinoxaline), DMH1, derivatives thereof, and the like. The AlK5 inhibitor II can be favorably used. When the ALK5 inhibitor II is used as the TGF-β inhibitor, the concentration in the culture medium is typically 0.1 µM to 50 µM and preferably 1 µM to 20 µM.

The concentration of nicotinamide used in the step D) in the culture medium is typically 1 mM to 100 mM and preferably 5 mM to 50 mM.

The culture in the step D) may be conducted in the presence of a ROCK inhibitor on the first day and conducted using a culture medium containing no ROCK inhibitor from the second day forward. The ROCK inhibitor may be the same as the ROCK inhibitor used in the production method of the present invention. When Y-27632 is used as the ROCK inhibitor, the concentration in the culture medium is typically 1 to 20 µM and preferably 5 to 15 µM.

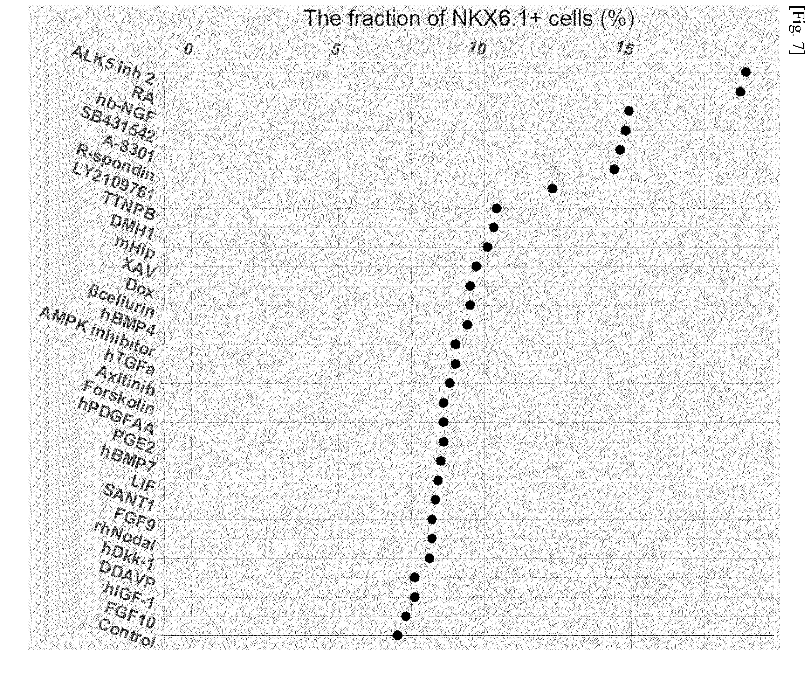

As shown in the section "Examples" below, it was shown that pancreatic endoderm cells were proliferated with improved efficiency by culturing the cells in the presence of a specific low-molecular-weight compound or protein (which may also be referred to as an "expansion culture promoter" hereinafter). Accordingly, the production method of the present invention may include a step of culturing pancreatic endoderm cells in a culture medium containing an expansion culture promotor. Examples of such an expansion culture promotor include a TGF-β inhibitor, a retinoic acid-receptor agonist, a growth factor (e.g., NGF, β cellurin, TGF-α, PDGFAA, LIF, IGF-1, FGF9, or FGF10), a bone morphogenetic protein (BMP) (e.g., BMP4 or BMP7), and the like, among which the TGF-β inhibitor and the retinoic acid-receptor agonist are preferable. More specific examples of the expansion culture promotor are shown in FIG. 7. Only one of the expansion culture promotors may be used, or two or more of them may be used. In an aspect of the present invention, the culture medium used in the production method of the present invention contains the TGF-β inhibitor and/or the retinoic acid-receptor agonist, but it preferably contains both the TGF-β inhibitor and the retinoic acid-receptor agonist.

The TGF-β inhibitor may be the same as the TGF-β inhibitor used in the step D). Among the TGF-β inhibitors, the ALK5 inhibitor II, SB431542, A-83-01, LY2109761, and DMH1 are preferable, and the ALK5 inhibitor II is more preferable. When the ALK5 inhibitor II is used as the TGF-β inhibitor, the concentration in the culture medium is typically 0.1 µM to 50 µM and preferably 1 µM to 20 µM. In an aspect, the concentration is 10 µM.

The retinoic acid-receptor agonist may be the same as the retinoic acid-receptor agonist used in the step C). Among the retinoic acid-receptor agonists, retinoic acid and TTNPB are preferable, and retinoic acid is more preferable. When retinoic acid or a salt thereof is used as the retinoic acid-receptor agonist, the concentration in the culture medium is typically 100 nM to 10 µM and preferably 500 nM to 5 µM. In an aspect, the concentration is 1 µM.

In another aspect of the present invention, pancreatic endoderm cells obtained using the production method of the present invention (such pancreatic endoderm cells are also referred to as "pancreatic endoderm cells of the present invention" hereinafter) are also provided. These pancreatic endoderm cells have at least a potential to differentiate into β-like cells and express at least PDX1 and NKX6.1. The pancreatic endoderm cells may further express one or more gene markers such as SOX9 and GATA4.

When the wording ""express" various gene markers such as PDX1" or ""positive" for the marker" is used in this specification, it implicates at least "production of mRNA encoded by the gene" unless otherwise stated, and preferably further implicates "production of a protein encoded by the mRNA". Therefore, when the production of mRNA encoded by a gene is detected through at least quantitative RT-PCR, it can be considered that the cells express the gene. Meanwhile, when the production of mRNA encoded by a gene is not detected through quantitative RT-PCR (i.e., the production level is below the detection limits) or is at the background level, it is considered that the cells do not express the gene or are negative.

### 2. Kit for Expansion Culture of Pancreatic Endoderm Cells

The present invention further provides a kit for expansion culture of pancreatic endoderm cells that includes a ROCK inhibitor and KGF and/or EGF (this kit may be referred to as an "expansion culture kit of the present invention" hereinafter). The "kit for expansion culture of pancreatic endoderm cells" can be referred to as a "kit for proliferation of pancreatic endoderm cells" or "kit for production of pancreatic endoderm cells". It is preferable that the expansion culture kit of the present invention includes nicotinamide. It is also preferable that the expansion culture kit of the present invention includes a TGF-β inhibitor and/or a retinoic acid-receptor agonist.

The expansion culture kit of the present invention may include a basal culture medium, additives for a culture medium, a culture vessel, and at least one type of pancreatic endoderm cells and progenitor cells thereof (e.g., pluripotent stem cells, embryonic endoderm cells, primitive gut tube cells, and posterior foregut cells). All the contents described in "1. Method for Producing Pancreatic Endoderm Cells" above are applied to the definitions, specific examples, and the like of the constituents such as a ROCK inhibitor, a TGF-β inhibitor, and a retinoic acid-receptor agonist included in the expansion culture kit of the present invention.

### 3. Method for Producing β-like Cells

As described above, the pancreatic endoderm cells of the present invention have at least a potential to differentiate into β-like cells. Accordingly, in another aspect, provided are a method for producing β-like cells or progenitor cells thereof that includes a step of inducing differentiation of the pancreatic endoderm cells of the present invention into β-like cells or progenitor cells thereof (this method may be referred to as a "method for producing β-like cells according to the present invention" hereinafter"), and β-like cells or progenitor cells thereof obtained using this method (these cells may also be referred to as "β-like cells of the present invention" hereinafter). Examples of the progenitor cells of β-like cells include endocrine cells expressing NGN3, immature β cells expressing insulin and NKX6.1, and the like. From the viewpoint of a clinical application, it is preferable to conduct the method for producing β-like cells according to the present invention under feeder-free conditions and xeno-free conditions throughout the entire process.

The differentiation of the pancreatic endoderm cells into β-like cells or progenitor cells thereof can be induced using known methods such as the methods described in Patent Literature 1, Non Patent Literature 1, and Non Patent Literature 2. Specifically, for example, the differentiation can be induced using a method that includes:
Step E) a step of inducing differentiation of the pancreatic endoderm cells of the present invention into endocrine progenitor cells; and
Step F) a step of inducing differentiation of the endocrine progenitor cells into β-like cells,
and the like.

A basal culture medium and additives for a culture medium used in the steps E) and F) may be the same as the basal culture medium and the additives for a culture medium used in the production method of the present invention. In the steps E) and F), the culture temperature is typically about 30°C to 40°C and preferably about 37°C. The culture is conducted under a CO₂-containing air atmosphere, and the CO₂ concentration is preferably about 2% to 5%. In the steps E) and F), it is preferable to conduct suspension culture. The contents described in "1. Method for Producing Pancreatic Endoderm Cells" above are applied to the definition and method of the suspension culture.

In the step E), the differentiation into endocrine progenitor cells can be conducted by, for example, culturing the pancreatic endoderm cells of the present invention in a culture medium containing a γ-secretase inhibitor and a TGF-β inhibitor. The culture medium may also contain a thyroid hormone, a growth factor, a Hedgehog pathway inhibitor, a retinoic acid derivative, a BMP inhibitor, and the like. The culture period is typically 1 day to 5 days.

The contents described in the steps C) and D) above are applied to the types and concentrations of the TGF-β inhibitor, the growth factor, the Hedgehog pathway inhibitor, the retinoic acid derivative, and the BMP inhibitor used in the step E).

Examples of the γ-secretase inhibitor used in the step E) include RO4929097, DAPT (GSI-IX), Semagacestat (LY450139), Dibenzazepine (YO-01027), and the like. Among these, RO4929097 is preferable. When RO4929097 is used, the concentration in the culture medium is typically 0.1 to 10 µM and preferably 0.5 to 5 µM.

Examples of the thyroid hormone used in the step E) include triiodothyronine (T3), thyroxine (T4), and the like. Among these, triiodothyronine is preferable. When triiodothyronine is used, the concentration in the culture medium is typically 0.1 to 10 µM and preferably 0.5 to 5 µM.

In the step F), the differentiation into β-like cells can be conducted by, for example, culturing the endocrine progenitor cells obtained in the step E) in the same culture medium as used in the step E), except that the γ-secretase inhibitor is not contained. The culture period is typically 4 days to 10 days.

The contents described in "1. Method for Producing Pancreatic Endoderm Cells" above are applied to the other culture conditions, culturing method, additives for a culture medium, specific examples of the culture vessel, way to process the surface of the culture vessel, and the like.

It is also possible to produce pancreatic exocrine cells from the pancreatic endoderm cells of the present invention using a method known per se. An example of such a method is the method described in WO 2014/104403, and specifically, it is also possible to produce the pancreatic exocrine cells by conducting a step of culturing the pancreatic endoderm cells of the present invention in a culture medium to which a histone deacetylase inhibitor and/or a Notch signaling ligand protein, and a protein kinase C activator have been added. All the contents of WO 2014/104403 are applied to the specific types of histone deacetylase inhibitor, Notch signaling ligand protein, and protein kinase C activator, and the production method.

### 4. Agent for Cell Transplantation Therapy

Transplanting the pancreatic endoderm cells of the present invention and the β-like cells of the present invention (these cells may be collectively referred to as "cells of the present invention") into a living mammal makes it possible to induce differentiation into pancreatic islet-like cells. Accordingly, the cells of the present invention can be favorably used in a cell transplantation therapy, and therefore, in another aspect of the present invention, an agent for cell transplantation therapy, containing the cells of the present invention (this agent may be referred to as an "agent for cell transplantation therapy of the present invention" hereinafter) is provided. In addition, the present invention also encompasses a method for treating a pancreatic disease that includes administering or transplanting an effective amount of the cells of the present invention to a mammal (e.g., a human, a mouse, a rat, a monkey, a cow, a horse, a pig, or a dog) to be treated. Examples of the pancreatic disease to be treated include acute pancreatitis, chronic pancreatitis, type 1 diabetes, type 2 diabetes, pancreatic tumor, tumor of the Langerhans' islands, and the like.

The cells or agent for cell transplantation therapy of the present invention can be used for transplantation into the body of a patient in need thereof. It is preferable to conduct the transplantation in a region in the living body where the cells can be fixed at a certain position, and the transplantation can be conducted under the skin, in the abdominal cavity, on the peritoneal epithelium, on the greater omentum, in the adipose tissue, in the muscular tissue, under the tunic of an organ such as the pancreas or the kidney, and the like. The subcutaneous transplantation, which is minimally invasive transplantation, is preferable. It is sufficient that a therapeutically-effective amount of cells to be transplanted is administered, and the amount may vary depending on the age, the weight, the size of the transplantation site, the disease severity, or the like of a transplantation target and is not particularly limited, but is, for example, about 10×10⁴ cells to 10×10¹¹ cells.

When the cells of the present invention are used in cell transplantation therapy, it is desirable to use cells derived from iPS cells established from somatic cells with the same or substantially the same HLA genotype as that of a transplantation target individual from the viewpoint of preventing rejection. Here, "substantially the same" means that the HLA genotypes are identical to an extent that the immunological reaction to the transplanted cells can be suppressed using an immunosuppressive agent, and, for example, somatic cells with the HLA type in which three genetic loci (HLA-A, HLA-B, and HLA-DR) are respectively identical or four genetic loci (HLA-C in addition to the three loci above) are respectively identical are used. When a sufficient amount of cells cannot be obtained due to the age, diathesis, or the like, the cells can be embedded in a capsule made of polyethylene glycol or silicone, a porous vessel, or the like for the purpose of avoiding rejection and then transplanted.

A parenteral preparation such as an injection, a suspension, or a drip is produced by, for example, mixing the cells of the present invention with a pharmaceutically acceptable carrier in accordance with a conventional procedure. Accordingly, in an aspect, a method for producing an agent for cell transplantation therapy is also provided, the method including a step of formulating the cells of the present invention. This production method may include a step of preparing the cells of the present invention. In addition, the production method can also include a step of preserving the cells of the present invention.

Examples of the pharmaceutically acceptable carrier that can be contained in the parenteral preparation include aqueous liquids for injection such as a physiological saline solution and an isotonic solution containing glucose and other adjuvants (e.g., D-sorbitol, D-mannitol, and sodium chloride). The agent for cell transplantation therapy of the present invention may be blended with a buffering agent (e.g., a phosphate buffer solution or sodium acetate buffer solution), a soothing agent (e.g., benzalkonium chloride or procaine hydrochloride), a stabilizer (e.g., human serum albumin or polyethylene glycol), a preservative, an antioxidant, and the like.

The agent for cell transplantation therapy of the present invention can be used as follows: it is provided in a state of being cryopreserved under the conditions commonly used for cryopreservation of cells, and is thawed before use. In such a case, the agent for cell transplantation therapy may further contain serum or a substitute thereof, an organic solvent (e.g., DMSO), and the like. In such a case, the concentration of the serum or the substitute thereof is not particularly limited, but can be about 1% to about 30% (v/v) and preferably about 5% to about 20% (v/v). The concentration of the organic solvent is not particularly limited, but can be 0% to about 50% (v/v) and preferably about 5% to about 20% (v/v).

Hereinafter, the present invention will be described more specifically by way of examples, but the present invention is not limited to these examples by any means.

### Examples

### <Materials and Methods>

### Screening of Senescence-Related Reagents

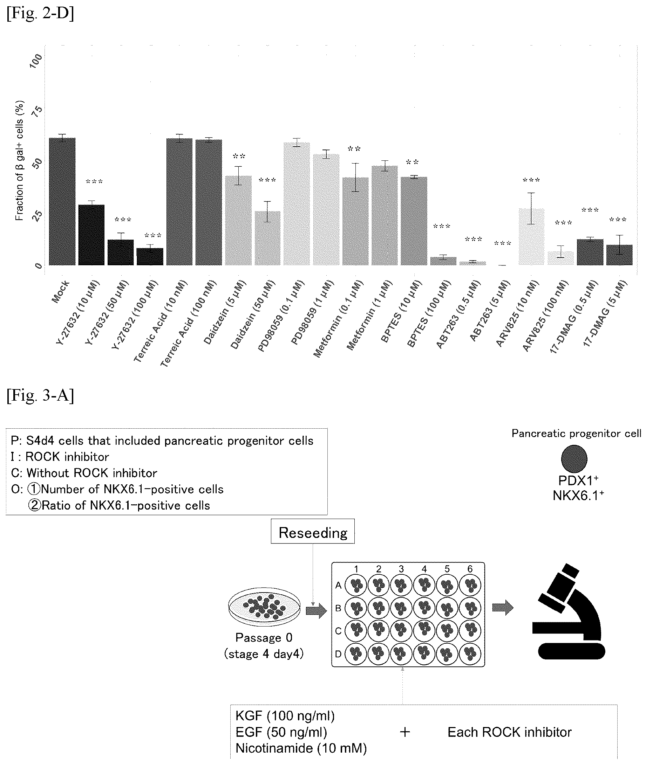

Cells were differentiated to a pancreatic endoderm cell level using a stepwise differentiation-inducing method, and were then re-seeded on a 24-well plate at 2×10⁵/well. The following culture media were added to these cultured cells, and the cells were cultured for 1 week. The culture medium for the Control group was improved MEM to which 0.5×B-27 Supplement, 100 U/ml penicillin/streptomycin, 100 ng/ml KGF/FGF7, 50 ng/ml EGF, and 10 mM nicotinamide had been added. For the Intervention group, a culture medium obtained by adding Y-27632, terreic acid, Daidzein, PD98059, Metformin, BPTES, ABT263, ARV825, or 17-DMAG to the culture medium above was added, and the cells were cultured for 1 week in the same manner. After fixed using 4% PFA, the cells were subjected to β-gal staining and NKX6.1 antibody immunostaining, and fluorescently stained images were acquired using BZ-800 (Keyence). The number of NKX6.1-positive cells, the NKX6.1-positive ratio, and the β-gal-positive ratio were calculated using the BZ-H4CM/Macro Cell Count function of that microscope. FIGS. 2-A and 3-A show the outline of this procedure.

### Screening of Expansion Culture Promoting Reagents

The cells on Day 21 of expansion culture were cultured for 6 days using a culture medium obtained by adding various growth factors and small-molecule compounds to a culture medium for expansion culture containing Y-27632. The other conditions were the same as those of the screening of senescence-related reagents.

### Expansion Culture of PDX1⁺/NKX6.1⁺ Pancreatic Endoderm Cells

### 1. Production of PDX1⁺/NKX6.1⁺ Pancreatic Endoderm Cells

PDX1⁺/NKX6.1⁺ pancreatic endoderm cells were produced from iPS cells (585A1 line) or ES cells (KhES-3 line) on a 6-well plate using the method by Toyoda et al. (Toyoda T, et al. Stem Cell Reports 2017). Briefly, the undifferentiated cells above were seeded at 1×10⁶/well on a 6-well plate coated with iMatrix-511 silk. The cells were cultured in RPMI to which an S1 culture medium (1×B-27 Supplement, 100 U/ml penicillin/streptomycin, 100 ng/ml activin A, and CHIR99021 (Day 1: 3 µM, Days 2 and 3: 1 µM, and Day 4: 0 µM)) had been added for 4 days, in an S2 culture medium (improved MEM to which 0.5×B-27 Supplement, 100 U/ml penicillin/streptomycin, and 50 ng/ml KGF/FGF7 had been added) for 5 days, and in an S3 culture medium (improved MEM to which 0.5×B-27 Supplement, 100 U/ml penicillin/streptomycin, 50 ng/ml KGF/FGF7, 0.2 µM LDN-193189, 0.1 µM SANT-1, and 10 nM TTNPB had been added) for 2 days. The cells were detached from the 6-well plate using trypsin, and were reseeded on a 6-well plate coated with iMatrix-511 silk at 1.5×10⁶/well using the S3 culture medium again. On the next day, the culture medium was changed to an S4 culture medium (improved MEM to which 0.5×B-27 Supplement, 100 U/ml penicillin/streptomycin, 100 ng/ml KGF/FGF7, 50 ng/ml EGF, 10 mM nicotinamide, and 50 µM Y-27632 had been added), and the cells were cultured for 4 days.

### 2. Expansion Culture of PDX1⁺/NKX6.1⁺ Pancreatic Endoderm Cells

(1) After the cells produced in clause 1 above were washed with PBS(-), 1 ml of 0.25% trypsin-EDTA was added thereto, and the cells were incubated at 37°C and 5% CO₂ for 5 minutes. (2) The adherent cells were detached through pipetting and were added to a 50-ml centrifuge tube. (3) 4 ml of DMEM (Dulbecco's Modified Eagle Medium) /10% FBS / PS (penicillin-streptomycin) was added to the centrifuge tube. (4) The resulting mixture was centrifuged at 400 G for 3 minutes, and the supernatant was removed. (5) The cells were suspended in improved MEM (containing 10 µM Y-27632, 0.5×B-27 Supplement, and 100 U/ml penicillin/streptomycin), and were counted. (6) When the cells were seeded on a 6-well plate, an amount of the suspension necessary to provide 1×10⁶ cells/well was collected and was centrifuged at 400 G for 3 minutes (2×10⁵ cells/well for a 24-well plate). (7) After the supernatant was removed, the cells were re-suspended in improved MEM (containing 100 ng/ml KGF, 50 ng/ml EGF, 10 mM nicotinamide, 50 µM Y-27632, 0.5×B-27 Supplement, and 100 U/ml penicillin/streptomycin), the suspension was adjusted to correspond to 2 ml/well, and then the cells were seeded. A 6-well plate to which the cells were to be seeded had been coated in advance by adding a solution formed by mixing 10 µl of iMatrix-511 silk and 1.5 ml of PBS(-) to each well 1 hour before seeding, and incubating the plate at 37°C and 5% CO₂ for 1 hour.

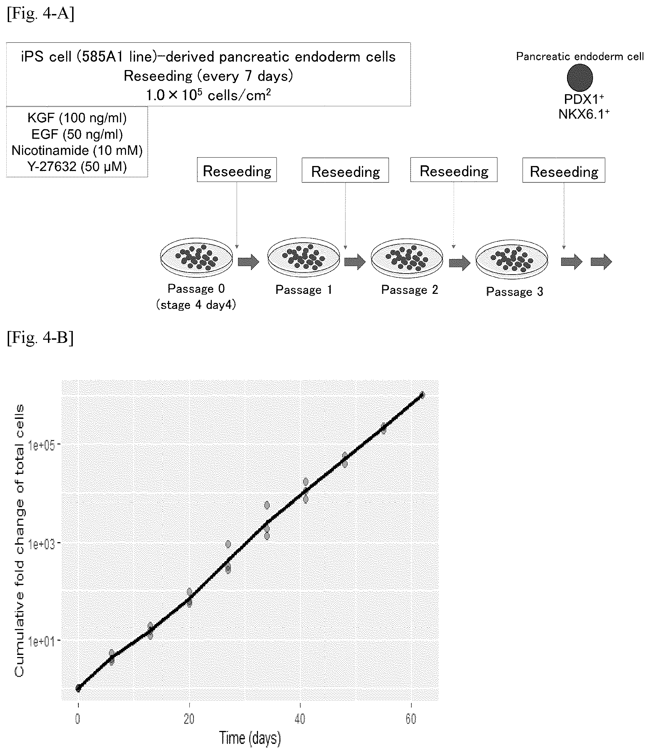

(8) On the next day (Day 1), the cells were washed with improved MEM, and then the culture medium was changed to improved MEM (5 ml/well) containing 100 ng/ml KGF, 50 ng/ml EGF, 10 mM nicotinamide, 50 µM Y-27632, 0.5× B-27 Supplement, and 100 U/ml penicillin/streptomycin. (9) The cells were washed with improved MEM 3 days after that (on Day 4), and then the culture medium was changed to the same culture medium as that in the step (7) (5 ml/well). (10) The steps (1) to (7) above were conducted 3 days after that (on Day 7), and the next passage (expansion culture) was conducted. Expansion culture and passage culture were repeated by repeating the steps (8), (9), and (10) from the next day. One round of the passage (expansion culture) corresponded to 7 days (operations above). FIGS. 4-A show the outline of this procedure. When an ALK5 inhibitor (Fujifilm Wako Chemicals; 018-23023; ALK5 inhibitor II (CAS: 446859-33-2)) (the concentration in the culture medium: 10 µM) and retinoic acid (Sigma; R2625; CAS: 302-79-4)) (the concentration in the culture medium: 1 µM) were used, the ALK inhibitor and RA or DMSO (the concentration in the culture medium: 0.1% (v/v)) had been added in advance to the culture media used in the steps (7) to (9).

### Induction of Differentiation into β-like Cells

After the expansion culture, the differentiation into β-like cells was induced using the method by Kimura et al. (Non Patent Literature 1) with a portion thereof being modified. Briefly, pancreatic endoderm cells were detached from a 6-well plate using trypsin, and were then suspended in the S4 culture medium at 3×10⁵ cells/ml. The cells were seeded on a V-bottomed 96-well plate at 3×10⁴ cells/100 µl/well. After the cells were incubated at 37°C and 5% CO₂ for 1 day, the culture medium was changed to an S5 culture medium (improved MEM to which 0.5×B-27 Supplement, 100 U/ml penicillin/streptomycin, 10 µM ALK5 inhibitor II (CAS: 446859-33-2), 1 µM triiodothyronine (T3), 1 µM RO4929097, and 20 ng/ml Betacellulin had been added), and then the cells were cultured for 1 week. Thereafter, the culture medium was changed to an S6 culture medium (improved MEM to which 0.5×B-27 Supplement, 100 U/ml penicillin/streptomycin, 10 µM ALK5 inhibitor II, and 1 µM T3 had been added), and the cells were further cultured for 1 week.

### Evaluation Methods

### 1. Immunostaining

The cells after the expansion culture were washed with PBS(-) twice, and were then fixed using 4% PFA at 4°C for 20 minutes. The cells were blocked with a blocking solution (PBS(-) containing 5% donkey serum and 0.4% Triton X-100) at room temperature for 30 minutes. The cells were incubated in a primary antibody solution diluted with the blocking solution at 4°C overnight. After the primary antibody solution was washed off, the cells were incubated at room temperature for 1 hour in a fluorescent secondary antibody solution diluted with the blocking solution. Fluorescently immunostained images were acquired using BZ-710 or BZ-800 (Keyence).

### 2. Flow cytometry analysis

The cells after the expansion culture were detached using 0.25% trypsin-EDTA, and were then fixed using Cytofix/Cytoperm Kit (BD Biosciences) in accordance with the protocol. The cells were blocked with a permeabilization solution containing 2% donkey serum. The cells were incubated in a primary antibody solution diluted with a blocking solution at 4°C overnight. After the primary antibody solution was washed off, the cells were incubated at room temperature for 1 hour in a fluorescent secondary antibody solution diluted with the blocking solution. The stained cells were analyzed using FACSAria II (BD Biosciences).

### 3. Quantitative Evaluation of Cell Nuclear Morphology

The nuclei of the cells on a plastic bottom plate that had been fixed using 4% PFA were stained with Hoechst. From the fluorescently stained images acquired using BZ-800 (Keyence), the cell morphology evaluation values that included the eccentricity (circularity) were acquired using the Measure Object Size Shape function of the CellProfiller software.

### Statistical Analysis

The Mann-Whitney U test was used as a method for nonparametrically comparing two groups. One-way analysis of variance (one way ANOVA) was used to conduct comparison analysis of 3 or more groups. When a significant difference was shown in ANOVA, the Dunnett's method was used for multiple comparison between one control group and other treatment groups. It was defined that there was a significant difference when the P value was smaller than 0.05.

The following describes the information on the reagents and the like used in Examples.

**[Table 1-1]**

| **REAGENT or RESOURCE** | SOURCE | IDENTIFIER |
|---|---|---|
| Goat polyclonal anti-PDX1 | R&D | Cat# AF2419; RRID: AB_355257 |
| Mouse monoclonal anti-Ki67 | BD Biosciences | Cat# 556003; RRID: AB_396287 |
| Rabbit monoclonal anti-NKX6.1 | Cell Signaling Technology | Cat# 54551; RRID: AB_2722625 |
| Rat monoclonal anti-C-peptide | DSHB | Cat# GN-ID4; RRID: AB_2255626 |
| Donkey anti-Goat IgG-Alexa Fluor 488 | Thermo Fisher Scientific | A11055 |
| Donkey anti-Goat IgG-Alexa Fluor 546 | Thermo Fisher Scientific | A11056 |
| Donkey anti-Goat IgG-Alexa Fluor 647 | Thermo Fisher Scientific | A21447 |
| Donkey anti-Mouse IgG-Alexa Fluor 488 | Thermo Fisher Scientific | A21202 |
| Donkey anti-Mouse IgG-Alexa Fluor 546 | Thermo Fisher Scientific | A10036 |
| Donkey anti-Mouse IgG-Alexa Fluor 647 | Thermo Fisher Scientific | A31571 |
| Donkey anti-Rabbit IgG-Alexa Fluor 488 | Thermo Fisher Scientific | A21206 |
| Donkey anti-Rabbit IgG-Alexa Fluor 546 | Thermo Fisher Scientific | A10040 |
| Donkey anti-Rabbit IgG-Alexa Fluor 647 | Thermo Fisher Scientific | A31573 |
| | | |
| iMatrix-511 silk | Nippi | 892021 |
| improved MEM | Thermo Fisher Scientific | 10373-017 |
| B-27 Supplement | Thermo Fisher Scientific | 17504044 |
| Penicillin/Streptomycin | Fujifilm Wako Chemicals | 168-23191 |
| Y-27632 | Fujifilm Wako Chemicals | 251-00514 |
| KGF/FGF7 | R&D | 251-KG |
| EGF | R&D | 236-EG |

**[Table 1-2]**

| | | |
|---|---|---|
| Nicotinamide | STEMCELL Technologies | 7154 |
| Hoechst 33342 | Thermo Fisher Scientific | H3570 |
| ALK5 inhibitor | Fujifilm Wako Chemicals | 018-23023 |
| Triiodothyronine | Sigma-Aldrich | 64245 |
| RO4929097 | Selleck Chemicals | S1575 |
| Recombinant Human Betacellulin Protein | R&D | RSD-261-CE |
| Terreic Acid | Santa cruz | sc-200655 |
| Daidzein | Sigma-Aldrich | D7802 |
| PD 98059 | Sigma-Aldrich | P215 |
| Metformin | Pfizer | 4987-114-12280-3 |
| BPTES | Sigma-Aldrich | SML0601 |
| ARV825 | Medchem Express | HY-16954 |
| ABT263 | Medchem Express | HY-10087 |
| 17-DMAG | Selleck Chemicals | S1142 |
| FBS | biosera | FB-1285/500 |

### Example 1: Search for Reagent Enabling Proliferation of Pancreatic Endoderm Cells

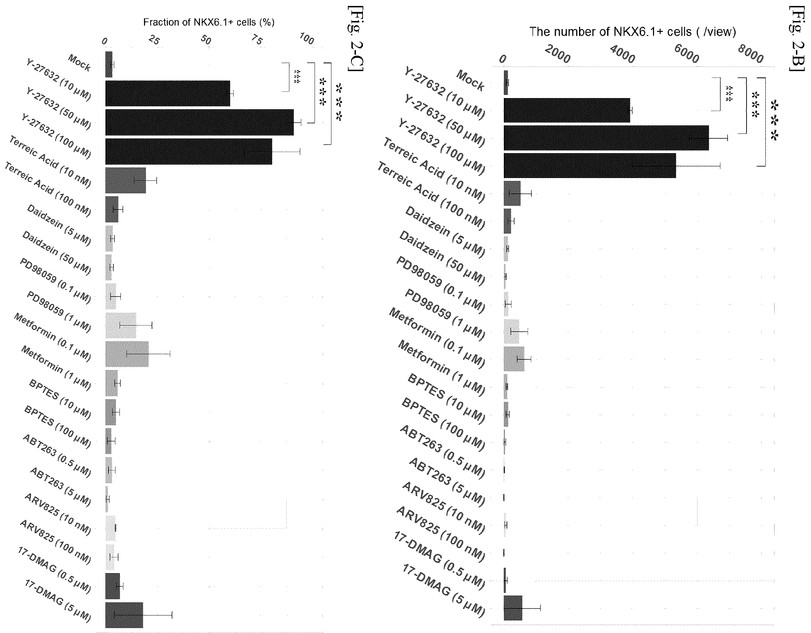

A reagent enabling proliferation of pancreatic endoderm cells derived from human iPS cells was searched for in the senescence-related reagents. Y-27632, terreic acid, Daidzein, PD98059, Metformin, BPTES, BPTES, ABT263, ARV825, and 17-DMAG were selected as the senescence-related reagents, and S4d4 cells that included NKX6.1⁺ pancreatic endoderm cells were cultured in a culture medium containing each of the senescence-related reagents. Then, (1) the number of NKX6.1-positive cells, or (2) the ratio of NKX6.1-positive cells and the ratio of β gal-positive cells were measured after the culture. FIGS. 2-B to 2-D show the results. FIG. 2-B shows that Y-27632 significantly increased the number of the target cells and the concentration of Y-27632 that was the most suitable for the proliferation was 50 µM, whereas other senescence-related reagents did not significantly increase the number of the pancreatic endoderm cells. FIG. 2-C shows that, even when the outcome was the ratio of the pancreatic endoderm cells, 50 µM of Y-27632 was the most effective as in FIG. 2-B. Also, FIG. 2-D shows that Y-27632 also exhibited an effect of reducing the ratio of β gal-positive cells (senescent cells). Meanwhile, the other senescence-related reagents also reduced the ratio of β gal-positive cells, but, considering FIG. 2-B as well, it is inferred that the reduction in the ratio of β gal-positive cells caused by BPTES, ABT263, and the like resulted from their toxicity.

### Example 2: Examination of Effect of Proliferating Pancreatic Endoderm Cells Exhibited by Rock Inhibitor Other than Y-27632

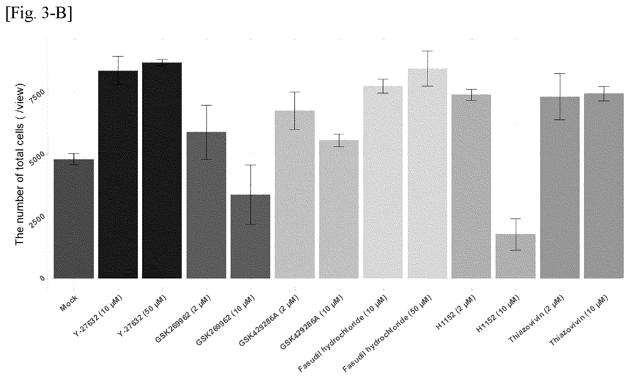

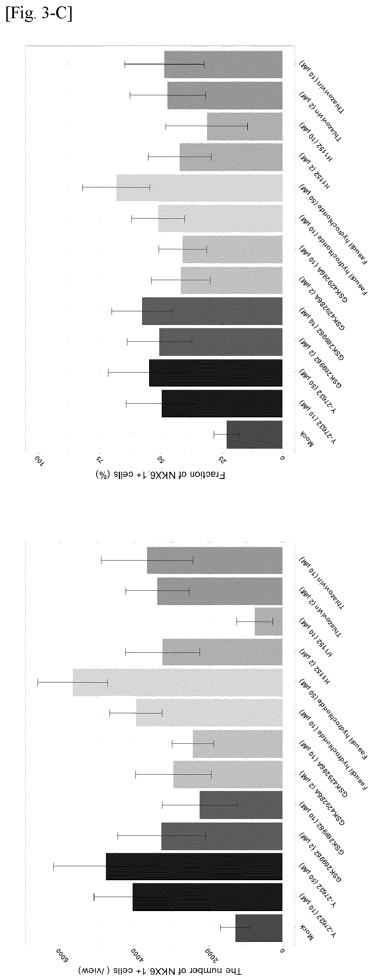

Since it had been confirmed in Example 1 that Y-27632, a ROCK inhibitor, exhibited an effect of proliferating pancreatic endoderm cells, it was examined whether or not other ROCK inhibitors would also exhibit an effect of proliferating PDX1⁺/NKX6.1⁺ pancreatic endoderm cells derived from human iPS cells. The experiments were conducted in the same manner as in Example 1. FIGS. 3-B and 3-C show the results. It was confirmed from FIGS. 3-B and 3-C that, similarly to Y-27632, the ROCK inhibitors (GSK269962, GSK429286A, Fasudil hydrochloride, H1152, and Thiazovivin) other than Y-27632 also exhibited the effect of proliferating pancreatic endoderm cells.

The results above strongly suggest that a wide variety of ROCK inhibitors exhibit the effect of proliferating pancreatic endoderm cells irrespective of the type.

### Example 3: Examination of Expansion Culture of Pancreatic Endoderm Cells with Y-27632 (50 µM)

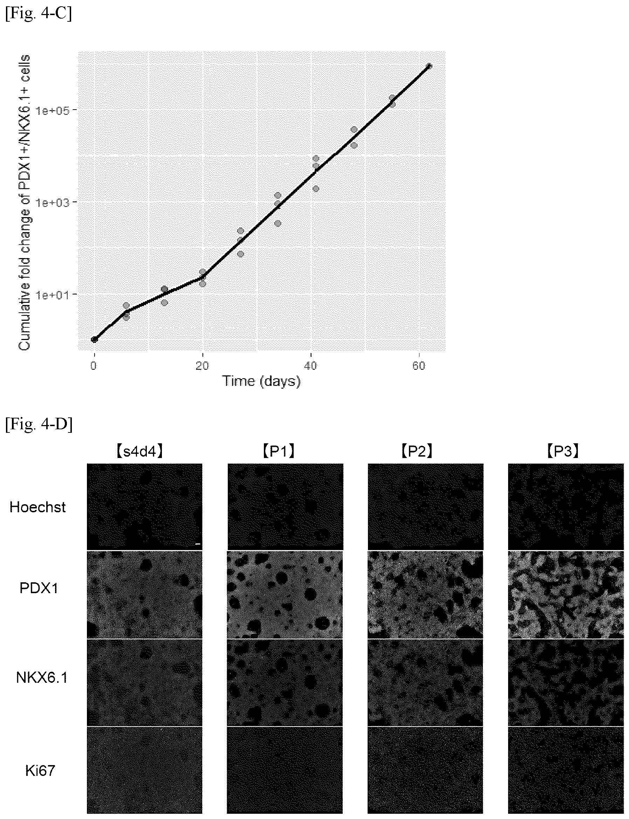

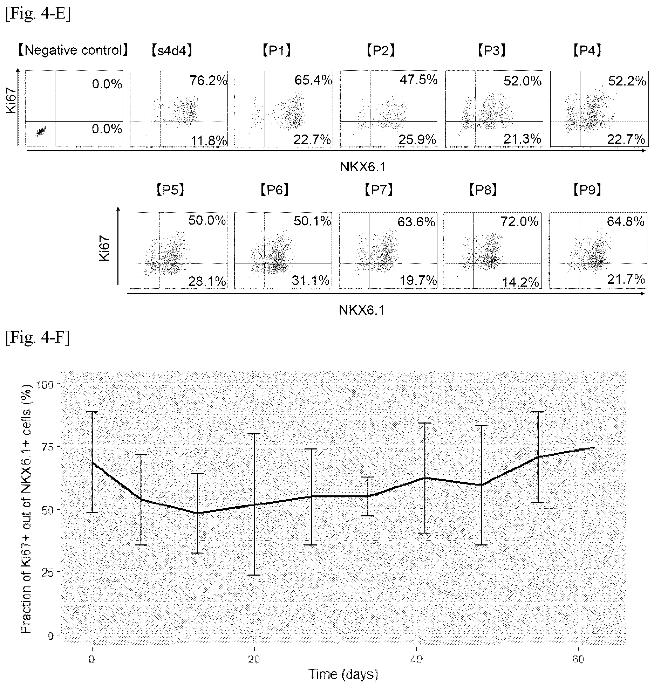

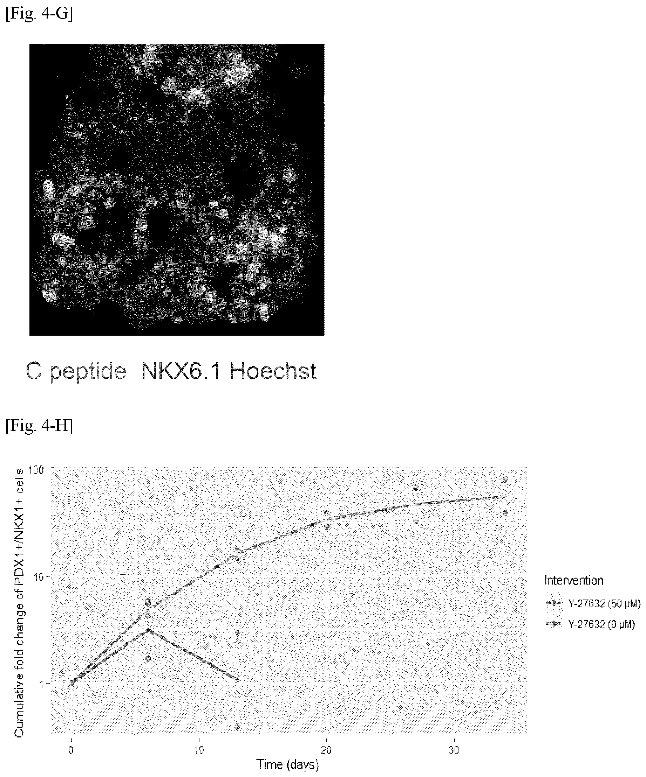

It was examined whether or not use of Y-27632 enabled expansion culture of pancreatic endoderm cells derived from human iPS cells. FIGS. 4-B to 4-H show the results. It was confirmed from FIG. 4-B that the total number of cells increased by a factor of 1×10⁵ or more for 60 days by conducting the expansion culture with Y-27632 (50 µM). It was confirmed from FIG. 4-C that the number of PDX1⁺/NKX6.1⁺ pancreatic endoderm cells increased by a factor of 1×10⁵ or more for 60 days by conducting the expansion culture with Y-27632 (50 µM). It was confirmed from the immunostained images of the cells after the expansion culture with Y-27632 (50 µM) in FIG. 4-D that the expression of PDX1 and NKX6.1 was maintained in the cells, and the expression of Ki67 was also maintained. It was confirmed from FIG. 4-E that the ratio of the pancreatic endoderm cells and the Ki67-positive ratio were kept due to the expansion culture with Y-27632 (50 µM). It was confirmed from FIG. 4-F that the proliferative capacity of the pancreatic endoderm cells (= the Ki67-positive ratio) was also maintained due to the expansion culture with Y-27632 (50 µM). It was confirmed from FIG. 4-G that even the pancreatic endoderm cells after 2 rounds of the expansion culture also had a potential to differentiate into β-like cells. It was confirmed from FIG. 4-H that, even when pancreatic endoderm cells derived from ES cells (KhES-3 line) were used, the expansion culture of the pancreatic endoderm cells was similarly possible, but the expansion culture was difficult without using Y-27632.

The results above show that using Y-27632 (50 µM) enables very efficient expansion culture of pancreatic endoderm cells for at least 60 days, and the pancreatic endoderm cells after the expansion culture retain a potential to differentiate into β-like cells.

### Example 4: Examination of Expansion Culture of Pancreatic Endoderm Cells with Y-27632 at Different Concentrations

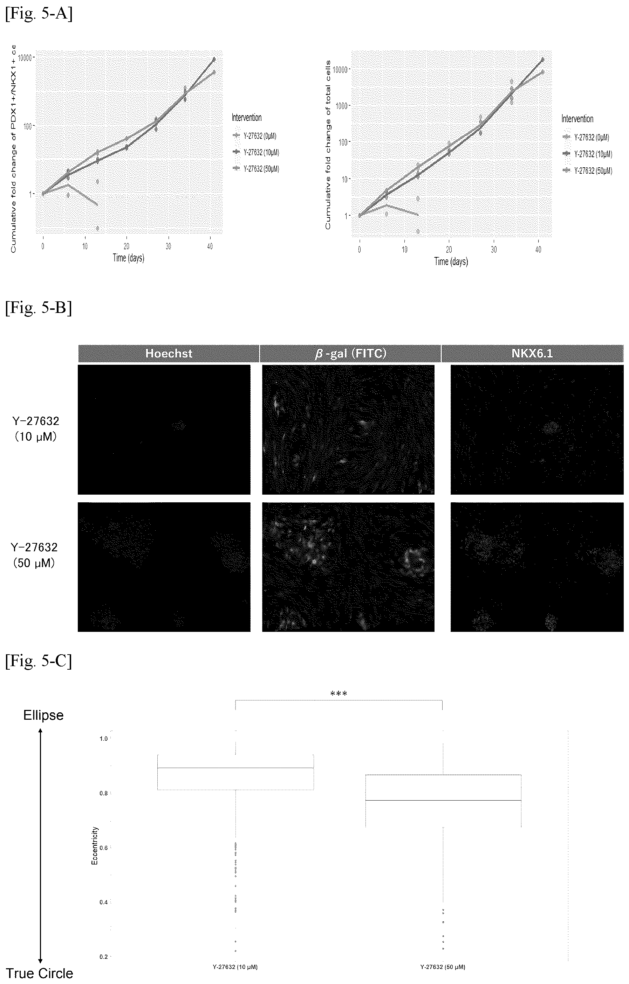

Since it had been demonstrated in Example 3 that using 50 µM of Y-27632 enabled the expansion culture of pancreatic endoderm cells, an influence of reducing the concentration to 10 µM on the expansion culture was examined. FIGS. 5-A to 5-C show the results. It was confirmed from FIG. 5-A that, even when Y-27632 (10 µM) was used, the number of PDX1⁺/NKX6.1⁺ pancreatic endoderm cells and the total number of cells increased by a factor of about 1×10⁴ for 40 days as in the case of using Y-27632 (50 µM). Meanwhile, it was shown in FIGS. 5-B and 5-C that, when Y-27632 (10 µM) was used, pancreatic endoderm cells derived from the KhES-3 line after passaged and cultured 5 times (5 weeks) changed their shape (elongated elliptical shape).

The results above show that although the expansion culture is possible even when the concentration of Y-27632 in the culture medium is at least 10 µM, 50 µM is preferable from the viewpoint of the cell quality.

### Example 5: Examination of Mechanism Enabling Expansion Culture of Pancreatic Endoderm Cells with Y-27632

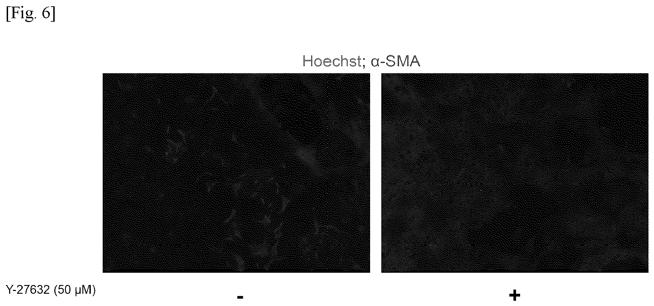

A mechanism by which the expansion culture of pancreatic endoderm cells with Y-27632 was enabled was examined. It was shown that, at the third passage of the expansion culture, the number of α-SMA-positive cells increased in the Y-27632 non-administration group compared with the group to which Y-27632 at a concentration of 50 µM was administered (FIG. 6). That is to say, it was observed that fibrosis or epithelial-mesenchymal transition was suppressed by administering Y-27632 to the cells.

The results above suggest that the main mechanism by which the expansion culture of pancreatic endoderm cells with Y-27632 was enabled is not based on an anti-apoptosis effect, but based on suppression of cellular senescence and the resulting suppression of fibrosis or epithelial-mesenchymal transition.

### Example 6: Search for Reagent Promoting Proliferation of Pancreatic Endoderm Cells

It was observed that, with the culture medium for expansion culture with Y-27632, the NKX6.1-positive ratio and the efficiency of induction to β-like cells tended to gradually decrease as the induction was repeated. Accordingly, an attempt was made to improve the expansion culture method by conducting screening using a low-molecular-weight compound and a protein such as a growth factor.

After passaged three times using a culture medium to which Y-27632 (50 µM) had been added, pancreatic endoderm cells were seeded on a 24-well plate coated with iMatrix at 2.0×10⁵ cells/well. Each of screening reagents was added to the culture medium containing Y-27632 (50 µM), and then the cells were cultured. After 6 days, the ratio of NKX6.1-positive cells was checked through cell immunostaining. As a result, the ALK5 inhibitor (ALK5i) and the retinoic acid-receptor agonist were identified as candidates for a factor for improving expansion culture (FIG. 7).

### Example 7: Examination of ALK5 Inhibitor (ALK5i) and Retinoic Acid-Receptor Agonist

It was tested whether or not expansion culture could be conducted for a long period of time while the NKX6.1-positive ratio was kept, by adding the two candidate factors to the expansion culture method with Y-27632 (50 µM).

Passages of the pancreatic endoderm cells were repeated every week, and the cells were counted. Immunostaining was simultaneously conducted each time, the NKX6.1-positive ratio was checked, and the cumulative NKX6.1 increase rate was calculated from the obtained values. This calculation method was conducted under two conditions, namely "DMSO group (Y-27632 + DMSO)" and "ALK5i + RA group (Y-27632 + ALK5i + RA)". As a result, the addition of ALK5i and RA improved the proliferation efficiency with the NKX6.1-positive ratio being kept compared with the Y-27632-alone group.

### Example 8: Examination of Efficiency of Induction to β-like Cells

In order to confirm the functions of pancreatic endoderm cells after expansion culture, the efficiency of induction into β-like cells after the expansion culture was checked.

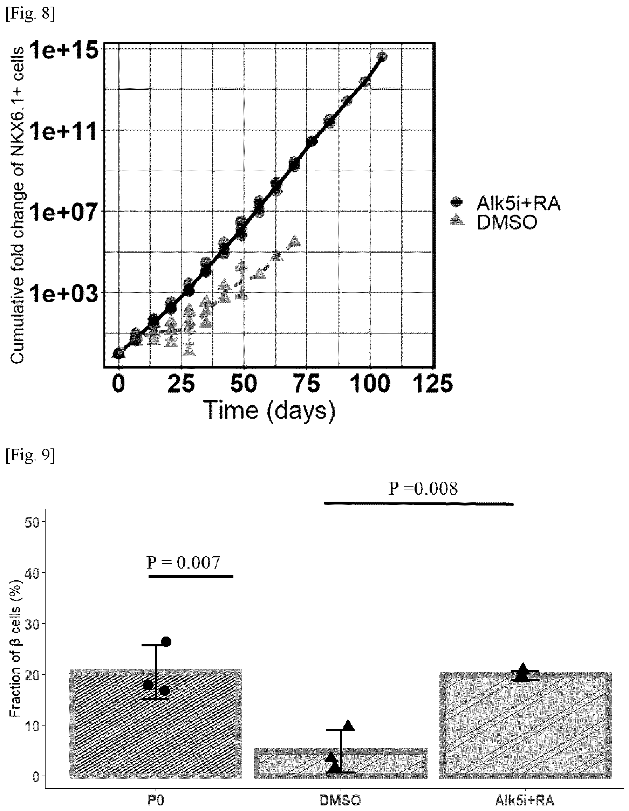

The induction of differentiation of pancreatic endoderm cells that had been repeatedly passaged 5 times using the expansion culture method with "Y-27632 + DMSO" or "Y-27632 + ALK5i + RA" into β-like cells was promoted. Then, the efficiency of induction into β-like cells was compared with that of a cell cluster obtained by inducing pancreatic endoderm cells that had not been passaged into β-like cells. As a result, with the improved expansion culture method ("Y-27632 + ALK5i + RA"), the efficiency of induction into β-like cells was higher compared with the unimproved expansion culture method ("Y-27632 + DMSO"), and was equal to the induction efficiency when pancreatic endoderm cells before the expansion culture were used (FIG. 9).

### Industrial Applicability

The method of the present invention, pancreatic endoderm cells produced in accordance with the present invention, or pancreatic endocrine cells such as β-like cells and pancreatic exocrine cells that are derived from the pancreatic endoderm cells can be applied to development of cell therapy for pancreatic diseases (particularly type 1 and type 2 diabetes), a drug discovery screening system for pancreatic diseases, a production of a pancreatic disease model using iPS cells derived from a patient with a hereditary pancreatic disease, and the like.

The present application claims priority based on Japanese Patent Application No. 2022-153013 filed in Japan (filing date: September 26, 2022), the disclosure of which is incorporated herein in its entirety by reference.

## Claims

1. A method for producing a pancreatic endoderm cell, comprising a step of culturing a pancreatic endoderm cell in a culture medium containing: a ROCK inhibitor; and KGF and/or EGF.

2. The method according to claim 1, wherein the culture is conducted for 2 days or more.

3. The method according to claim 1 or 2, wherein the ROCK inhibitor is selected from the group consisting of Y-27632, GSK269962, GSK429286A, Fasudil hydrochloride, H1152, and Thiazovivin.

4. The method according to claim 1 or 2, wherein the ROCK inhibitor is Y-27632.

5. The method according to any one of claims 1 to 4, wherein the culture medium contains a TGF-β inhibitor and/or a retinoic acid-receptor agonist.

6. The method according to claim 5, wherein the TGF-β inhibitor is 2-[3-[6-methylpyridin-2-yl]-1H-pyrazol-4-yl]-1,5-naphthyridine.

7. The method according to claim 5 or 6, wherein the retinoic acid-receptor agonist is retinoic acid.

8. The method according to any one of claims 1 to 7, wherein the pancreatic endoderm cell is derived from a pluripotent stem cell.

9. The method according to any one of claims 1 to 8, wherein the pancreatic endoderm cell is a cell derived from a patient with a hereditary pancreatic disease.

10. The method according to any one of claims 1 to 9, wherein the culture is conducted under feeder-free conditions.

11. A pancreatic endoderm cell produced by the method according to any one of claims 1 to 10.

12. A kit for expansion culture of a pancreatic endoderm cell, comprising: a ROCK inhibitor; and KGF and/or EGF.

13. The kit according to claim 12, comprising a TGF-β inhibitor and/or a retinoic acid-receptor agonist.

14. A method for producing a β-like cell or a progenitor cell thereof, comprising a step of inducing differentiation of the pancreatic endoderm cell according to claim 11 into a β-like cell or a progenitor cell thereof.

15. An agent for cell transplantation therapy, comprising the pancreatic endoderm cell according to claim 11 or a cell produced by the method according to claim 14.

16. The agent according to claim 15 for treating diabetes.
